# EUROPEAN PATENT APPLICATION

(11) **EP 4 670 778 A1**
(43) Date of publication of application: **31.12.2025**
(21) Application number: 25180833.3
(22) Date of filing: 04.06.2025
(51) Int. Cl.: A61N 1/04, A61B 5/391, A61B 5/00, A61N 1/36, G06N 3/08

(54) **MATERNAL MONITORING SYSTEMS AND METHODS**

(30) Priority: 28.06.2024 US 202418758661
(71) Applicant: GE Precision Healthcare LLC, Waukesha, WI 53188 (US)
(72) Inventor: BOGINENI, Kiran, Waukesha, 53188 (US); JONNADULA, Bharathkumar, Waukesha, 53188 (US); ALLEN, Lisa, Waukesha, 53188 (US); KAVOORI SETHUMADHAVAN, Nagarpiya, Waukesha, 53188 (US); FALK, Steven M., Waukesha, 53188 (US)
(74) Representative: Kilburn & Strode LLP

(57) **Abstract**

A system for monitoring and stimulating uterine muscles includes a plurality of surface electrodes configured to be disposed on an abdomen of a maternal patient and a control system. The control system is configured to obtain electromyography (EMG) data via the plurality of surface electrodes, detect at least one indicator of insufficient uterine activity based on the EMG data, and generate stimulation pulses between at least two of the plurality of surface electrodes in response to detecting the at least one indicator of insufficient uterine activity.

## Description

### BACKGROUND

The present disclosure generally relates to patient monitoring for maternal patients during antepartum, intrapartum, and/or postpartum stages and/or to systems for muscle stimulation of the uterine muscles.

Postpartum hemorrhage results in severe vaginal bleeding after childbirth and is one of the leading causes of maternal death. PPH can occur early postpartum (i.e., within the first 24 hours) or late postpartum (i.e. greater than or equal to 24 hours postpartum). The leading cause of PPH is uterine atony, which is the failure of the uterus to contract after giving birth. Other causes of PPH are known, which may be alternative causes or may occur in conjunction with uterine atony to lead to PPH. Trauma such as lacerations of the uterus, cervix, and/or vagina may cause or contribute to PPH. Retained placenta or clots may also cause or contribute to causing PPH. Further, various placenta related causes such as abnormal placentation, including Placenta Accreta Spectrum (PAS), pre-existing or acquired coagulopathy, uterine inversion, etc. may cause or contribute to causing PPH. Currently, prevention of PPH is attempted by measures such as routine administration of medication and avoiding birth trauma (e.g. via episiotomy). Signs of PPH may include dizziness, faintness, and blurred vision. However, PPH is difficult to predict and can occur in maternal patients without any risk factors.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the present disclosure, a system for monitoring and stimulating uterine muscles includes a plurality of surface electrodes configured to be disposed on an abdomen of a maternal patient and a control system. The control system is configured to obtain electromyography (EMG) data via the plurality of surface electrodes, detect at least one indicator of insufficient uterine activity based on the EMG data, and generate stimulation pulses between at least two of the plurality of surface electrodes in response to detecting the at least one indicator of insufficient uterine activity.

In one embodiment, the control system is further configured to determine a stimulation frequency, a stimulation amplitude, and a stimulation pulse duration based on the at least one indicator of insufficient uterine activity and/or the EMG data, and to generate the stimulation pulses based on the simulation frequency, the stimulation amplitude, and the pulse duration.

In another embodiment, stimulation pulses are generated between an upper electrode positioned on an upper portion of the maternal abdomen and a lower electrode positioned on a lower portion of the maternal abdomen.

In another embodiment, the stimulation pulses are generated between two surface electrodes positioned diagonally on the maternal abdomen, including an upper electrode positioned on an upper portion the maternal abdomen and a lower electrode positioned lower and towards patient-left side of the maternal abdomen compared to position of the upper electrode.

In another embodiment, the at least one indicator of insufficient uterine activity is based on at least one of a frequency of the EMG data during at least one uterine contraction, an amplitude of the EMG data during the at least one uterine contraction, a duration of the at least one uterine contraction, and a contraction frequency of the at least one uterine contraction.

In another embodiment, the at least one indicator of insufficient uterine activity is based on a comparison of the frequency of the EMG data and the amplitude of the EMG data to one or more frequency thresholds and amplitude thresholds associated with uterus health, a tired uterus condition, a presence of endometrial tissue, and invasive placentation.

In another embodiment, the control system is further configured to determine that a stimulation response of the patient's uterine muscle to the stimulation pulses is insufficient and indicates no uterine response, then cease stimulation and generate an alert of insufficient response.

In another embodiment, the control system is further configured to detect the at least one indicator of insufficient uterine activity based on a current labor phase of the maternal patient.

In another embodiment, the current labor phase of the maternal patient is an antepartum phase and, wherein the at least one indicator of insufficient uterine activity includes at least one of an EMG frequency being less than at least one antepartum threshold frequency and an EMG amplitude of less than an antepartum threshold amplitude measured during uterine contractions of the maternal patient.

In another embodiment, the current labor phase of the maternal patient is an intrapartum phase and, wherein the at least one indicator of insufficient uterine activity includes at least one of an EMG frequency being less than at least one intrapartum threshold frequency and an EMG amplitude of less than an intrapartum threshold amplitude measured during uterine contractions of the maternal patient.

In another embodiment, the labor phase of the maternal patient is a postpartum phase and, wherein the at least one indicator of insufficient uterine activity includes at least one of an EMG frequency being less than at least one postpartum threshold frequency and an EMG amplitude of less than a postpartum threshold amplitude measured during uterine contractions of the maternal patient.

In another embodiment, the system further includes an antepartum model being a trained machine learning model stored in memory and trained to detect insufficient uterine activity based on EMG data obtained during antepartum phase of labor, wherein the control system is configured to utilize the antepartum model during the antepartum phase of labor of the maternal patient to detect the at least one indicator of insufficient uterine activity based on the EMG data.

In another embodiment, the system further includes an intrapartum model being a trained machine learning model stored in memory and trained to detect insufficient uterine activity based on EMG data obtained during intrapartum phase of labor, wherein the control system is configured to utilize the intrapartum model during the intrapartum phase of labor of the maternal patient to detect the at least one indicator of insufficient uterine activity based on the EMG data.

In another embodiment, the system further includes a postpartum model being a trained machine learning model stored in memory and trained to detect insufficient uterine activity based on EMG data obtained during postpartum phase of labor, wherein the control system is configured to utilize the postpartum model during the postpartum phase of labor of the maternal patient to detect the at least one indicator of insufficient uterine activity based on the EMG data.

In another embodiment, the control system is configured to select and utilize at least one of an antepartum model, an intrapartum model, and an postpartum model to detect the at least one indicator of insufficient uterine activity based on the EMG data.

In another aspect of the present disclosure, a method of controlling a patient monitoring and stimulation system for a maternal patient includes obtaining electromyography (EMG) data via a plurality of surface electrodes configured to be disposed on an abdomen of a maternal patient, detecting at least one indicator of insufficient uterine activity based on the EMG data, and generating stimulation pulses between at least two of the plurality of surface electrodes in response to detecting the at least one indicator of insufficient uterine activity.

In one embodiment, the at least one indicator of insufficient uterine activity is based on a frequency of the EMG data during at least one uterine contraction, an amplitude of the EMG data during the at least one uterine contraction, a duration of the at least one uterine contraction, and/or a contraction frequency of the at least one uterine contraction.

In another embodiment, the at least one indicator of insufficient uterine activity includes at least one of an EMG frequency of less than a threshold frequency and an EMG amplitude of less than a threshold amplitude.

In another embodiment, the method further includes determining a stimulation frequency, a stimulation amplitude, and a stimulation pulse duration based on the indicator of insufficient uterine activity and/or the EMG data, and generating a first set of stimulation pulses based on the simulation frequency, the stimulation amplitude, and the pulse duration.

In another embodiment, the method further includes obtaining further EMG data via the plurality of surface electrodes during generation of the stimulation pulses, detecting a stimulation response based on the EMG data determining a stimulation adjustment based on the stimulation response, and generating a second set of stimulation pulses between the at least two of the plurality of surface electrodes based on the stimulation adjustment.

In another embodiment, the stimulation adjustment includes at least one of a different stimulation frequency, a different stimulation amplitude, and a different pulse duration from the simulation frequency, the stimulation amplitude, and the pulse duration of the first set of stimulation pulses.

In another embodiment, the method further comprises determining that the stimulation response is insufficient based on the EMG data, and then ceasing stimulation and generating an alert of insufficient response.

In another embodiment, the stimulation pulses are generated between an upper electrode positioned on an upper portion of the maternal abdomen and a lower electrode positioned on a lower portion of the maternal abdomen.

In another embodiment, the method further includes detecting the indicator of insufficient uterine activity based on a current labor phase of the maternal patient, wherein the current labor phase is one of an antepartum phase, an intrapartum phase, and a postpartum phase.

In another embodiment, the method further includes selecting one of an antepartum model, an intrapartum model, and a postpartum model based on the current labor phase of the maternal patient, and utilizing the selected model to detect the at least one indicator of insufficient uterine activity based on the EMG data.

In another embodiment, the method further includes utilizing an antepartum model to detect the indicator of insufficient uterine activity, wherein the antepartum model is a trained machine learning model stored trained to detect insufficient uterine activity based on EMG data obtained during antepartum phase of labor.

In another embodiment, the method further includes utilizing an intrapartum model to detect the indicator of insufficient uterine activity, wherein the intrapartum model is a trained machine learning model trained to detect insufficient uterine activity based on EMG data obtained during intrapartum phase of labor.

In another embodiment, the method further includes utilizing a postpartum model to detect the indicator of insufficient uterine activity, wherein the postpartum model is a trained machine learning model trained to detect insufficient uterine activity based on EMG data obtained during postpartum phase of labor.

In another embodiment, the method further includes selecting at least one threshold frequency and at least one threshold amplitude based on the current labor phase of the maternal patient, and detecting the at least one indicator of insufficient uterine activity based on a comparison of the EMG data to the at least one threshold frequency and the at least one threshold amplitude.

Various other features, objects, and advantages of the invention will be made apparent from the following description taken together with the drawings.

### BRIEF DESCRIPTION OF THE DRAWINGS

The present disclosure is described with reference to the following Figures.
FIG. 1 is an embodiment of a block diagram of a maternal monitoring and stimulation control system, in accordance with the present disclosure.
FIG. 2 is an embodiment of a block diagram of an ultrasound system, in accordance with aspects of the present disclosure.
FIGS. 3-5 are schematic diagrams of one embodiment of a transducer array, in accordance with aspects of the present disclosure.
FIG. 6A illustrates an exemplary ultrasound system worn on a maternal patient and comprising a belt having the transducer array in FIG. 3 disposed about the abdomen of the maternal patient, in accordance with aspects of the present disclosure.
FIG. 6B illustrates another exemplary ultrasound system worn on a maternal patient and comprising a patch having the transducer array in FIG. 3 disposed on the abdomen of a maternal patient, in accordance with aspects of the present disclosure.
FIG. 7 is a schematic diagram of a diagnostic system comprising part of the ultrasound system in accordance with aspects of the present disclosure.
FIG. 8 is a diagrammatic representation of a neural network classifier trained for detection of one or more PPH indicators in accordance with aspects of the present disclosure.
FIG. 9 is a diagrammatic representation of a construction of a neural network in accordance with aspects of the present disclosure.
FIG. 10 is a flow chart illustrating an exemplary method for monitoring a maternal patient for postpartum hemorrhage (PPH) in accordance with aspects of the present disclosure.
FIG. 11 is another flow chart illustrating an exemplary method for monitoring a maternal patient for postpartum hemorrhage (PPH) in accordance with aspects of the present disclosure.
FIG. 12 is a schematic diagram of electrodes and other sensors of an exemplary maternal monitoring and stimulation control system disposed on the abdomen of a maternal patient in accordance with aspects of the present disclosure.
FIG. 13 depicts an exemplary data system for generating a PPH risk index in accordance with aspects of the present disclosure.
FIG. 14 is a flow chart embodying an exemplary method for monitoring a maternal patient for risk of PPH in accordance with aspects of the present disclosure.
FIG. 15 is a schematic diagram of a plurality of surface electrodes disposed on the abdomen of a maternal patient in accordance with aspects of the present disclosure.
FIG. 16 is a perspective view of an exemplary embodiment of and EMG monitoring and stimulation system comprising a plurality of surface electrodes in accordance with aspects of the present disclosure.
FIG. 17 is a schematic diagram of another embodiment of a plurality of surface electrodes disposed on the abdomen of a maternal patient in accordance with aspects of the present disclosure.
FIG. 18 demonstrates exemplary EMG data obtained from a maternal patient in accordance with aspects of the present disclosure.
FIG. 19 is a graph illustrating exemplary stimulation pulses applied to a maternal abdomen to stimulate uterine muscles in accordance with aspects of the present disclosure.
FIG. 20 is a an exemplary data system for monitoring and stimulating uterine muscles in accordance with aspects of the present disclosure.
FIG. 21 illustrates exemplary steps for monitoring uterine activity in accordance with aspects of the present disclosure.
FIG. 22 illustrates exemplary steps for monitoring uterine activity in accordance with aspects of the present disclosure.
FIG. 23 illustrates exemplary steps for monitoring uterine activity in accordance with aspects of the present disclosure.
FIG. 24 illustrates one embodiment of a data system for identifying a stimulation condition and generating a stimulation in accordance with aspects of the present disclosure.
FIG. 25 a flow chart illustrating one embodiment of a method of controlling uterine muscle stimulation of a maternal patient in accordance with aspects of the present disclosure.
FIG. 26 is a flow chart illustrating an embodiment of a method of controlling uterine muscle stimulation of a maternal patient after childbirth in accordance with aspects of the present disclosure.
FIG. 27 is a flow chart illustrating an embodiment of a method of controlling uterine muscle stimulation of a maternal patient after childbirth in accordance with aspects of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

As used herein, the terms controller or module may refer to, be part of, or include an application-specific integrated circuit (ASIC), an electronic circuit, a combinational logic circuit, a field programmable gate array (FPGA), a processor (shared, dedicated, or group) that executes code, or other suitable components that provide the described functionality, or a combination of some or all of the above, such as in a system-on-chip. The terms controller or module may include memory (shared, dedicated, or group) that stores code executed by the processor. The term code, as used herein, may include software, firmware, and/or microcode, and may refer to programs, routines, functions, classes, and/or objects. The term shared, as used above, means that some or all code from multiple modules may be executed using a single (shared) processor. In addition, some or all code to be executed by multiple different processors may be stored by a single (shared) memory. The term group, as used above, means that some or all code comprising part of a single controller or module may be executed using a group of processors. Likewise, some or all code comprising a single controller or module may be stored using a group of memories.

Aspects of the disclosure are described herein in terms of functional and/or logical block components and various processing steps. It should be appreciated that such block components may be realized by any number of hardware, software, and/or firmware components configured to perform the specified functions. For example, an embodiment may employ various integrated circuit components, e.g., memory elements, digital signal processing elements, logic elements, look-up tables, or the like, which may carry out a variety of functions under the control of one or more processors or other control devices. In addition, those skilled in the art will appreciate that the present invention may be practiced in conjunction with any number of medical devices, including any number of different physiological data acquisition devices, and that the system described herein is merely one example application. The connecting lines shown in the various figures contained herein are intended to represent example functional relationships and/or physical couplings between the various elements. It should be noted that many alternative or additional functional relationships or physical connections may be present in a practical embodiment.

Postpartum hemorrhage (PPH) is a leading cause of maternal death around the world. The inventors have recognized that while risk factors for PPH are known, there does not exist a comprehensive system for evaluating multiple risk factors and identifying high risk patients. Risk factors include uterine atony, endometritis, Placenta Accreta Spectrum (PAS), and retained placental tissue, among others. However, diagnosis of PPH usually occurs after a patient has presented with heavy bleeding, that is after PPH has already began. Treatment for PPH often involves uterine massage, where the uterine muscle is stimulated through a massage technique administered by a clinician and/or surgical cauterization.

In view of the foregoing, the inventors have endeavored to build a system which proactively monitors maternal patients and identifies patients at a high risk of PPH. In one aspect of the disclosure, an ultrasound system is worn which periodically scans the maternal patient, including generating an ultrasound image of the entire uterus of the maternal patient. The image of the uterus is then compared to baseline images to identify the presence of one or more PPH indicators, including uterine atony, retained placental tissue, endometritis. The identification of one or more risk factors for PPH can alert caregivers and patients to the need for heightened monitoring and awareness of the risk of PPH and enable caregivers to perform early intervention to prevent PPH or lessen its effects.

Alternatively or in addition to the ultrasound system, the disclosed maternal patient monitoring systems and methods may include other monitoring modalities, such as cardiac inputs, electromyogram (EMG) inputs, clinician inputs, and/or inputs from the patient's health record. The cardiac inputs configured to provide inputs regarding the maternal patient's cardiac condition, such as blood pressure, heart rate, ECG data representing the maternal heart beat, pulse oximetry data, or the like. In one embodiment, the system includes EMG electrodes configured to be worn on the maternal patient's abdomen to generate EMG data representing the activity of the uterine muscle tissue. The EMG data may be utilized to determine a risk factor for PPH and/or to assess whether uterine activity is sufficient.

In some embodiments, the system may include one or more surface electrodes configured to be adhered on the maternal abdomen to record EMG potentials and/or to deliver stimulation pulses to stimulate the uterine muscle to contract. In various embodiments described herein, the system may be configured to perform uterine muscle stimulation before, during, or after labor, such as to induce labor contractions and/or to decrease the risk for and treat PPH. The system may be configured to utilize surface electrodes on the maternal abdomen to obtain EMG data and assess response of the uterine muscle to the stimulation. In some implementations, the system may be configured to utilize the muscle response to control future stimulation pulses. For example, the system may be configured to compare the EMG data representing the muscle response of the uterine muscle to one or more thresholds or model waveforms in order to determine and effectuate adjustments of the stimulation pulses to optimize the pulse magnitude, frequency, duration, etc. to achieve a desired outcome or behavior of the uterine muscle.

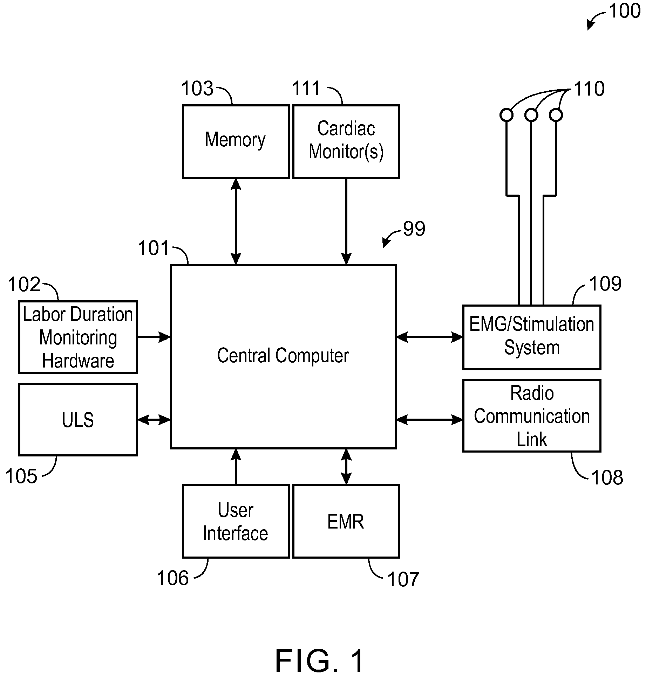

FIG. 1 depicts a schematic diagram of a maternal patient monitoring system 100 configured to assess a patient's risk for PPH, conduct patient monitoring during and after labor, and/or treat PPH through stimulation of the uterine muscle. The maternal monitoring system 100 includes an ultrasound system 105, an EMG monitoring and stimulation system 109, other labor monitoring hardware 102 configured to monitor other aspects of the maternal patient's labor, as well as other patient monitoring modalities including cardiac monitor(s) 111 configured to provide cardiac inputs, such as blood pressure, heart rate, SpO2, etc. The maternal monitoring system 100 is configured to obtain and process multiple information modalities regarding the maternal patient, including ultrasound images of the patient's uterus, EMG signals, cardiac information, labor information, information from the patient's medical record, and/or clinician inputs, to assess the maternal health, the risk of PPH, and/or the sufficiency of uterine activity during various phases of labor and/or after delivery.

In the depicted embodiment, the maternal monitoring system 100 comprises a central computer 101 configured to receive and process inputs from multiple elements, including ultrasound images of the uterus from the ultrasound system 105, EMG data from the EMG/stimulation system 109, clinician inputs from the user interface 106, labor monitoring information from the labor duration hardware 102, cardiac information from the cardiac monitor(s) 111, and/or patient medical history information from the patient's medical record housed in an electronic medical record (EMR) system 107. The central computer 101 comprises one or more programs configured to process these inputs, or at least a portion thereof, to perform various uterine and maternal health monitoring tasks, and may be configured to select and execute different programs depending on the stage of labor and delivery of the maternal patient (antepartum, intrapartum, or postpartum) and/or depending on the results of the monitoring modalities and the risks or conditions indicated thereby.

Ultrasound system 105, as described in more detail below, comprises an ultrasound transducer array configured to image the uterus of a maternal patient. In some implementations described herein, the ultrasound transducer array is configured to be positioned on the maternal abdomen and is sized to image the entire uterus without being repositioned or otherwise moved. The ultrasound system 105 includes an ultrasound control system configured to control the transducer array to automatically image the patient's uterus, such as at predetermined intervals, and to generate ultrasound images that can be processed by the system, such as the central computer 101, as described herein.

EMG/stimulation system 109 includes a plurality of surface electrodes 110 configured to be placed on the abdominal skin of the maternal patient. For example, the plurality of surface electrodes 110 may include at least three skin electrodes, but in some embodiments may include five or more electrodes distributed across the front of the maternal abdomen to record potentials generated due to contractions of the uterine muscle. Each of the plurality of electrodes 110 may be a separate element configured to adhere to the skin of the maternal patient via a conductive adhesive. Alternatively, the plurality of electrodes 110 may be integrated into a single patch configured to adhere to the maternal abdomen. The EMG/stimulation system 109 further comprises hardware configured to receive and process potentials recorded from between at least two of the electrodes to generate EMG data comprising EMG signals and/or values obtained therefrom, such as amplitude, frequency, and other values obtained by processing the EMG signals.

The EMG/stimulation system 109 may also include stimulation hardware configured to deliver a plurality of stimulation pulses via at least a subset of the plurality of EMG electrodes 110-i.e., to deliver a current at a predetermined voltage between at least two of the electrodes 110 on the maternal patient's abdomen. The stimulation pulses are configured to stimulate the maternal patient's uterine muscle, such as via a train of several pulses generated sequentially. The EMG/stimulation system 109 may be configured to deliver stimulation pulses and to monitor the response of the uterine muscle by record EMG data processing the EMG data to identify the aspects of the EMG signal representing the response.

Cardiac monitor(s) 111 includes hardware configured to sense a blood pressure of a maternal patient. For instance, cardiac monitor(s) 111 may comprise a blood pressure cuff system, such as is known in the art, for monitoring blood pressure. The blood pressure cuff system may comprise a cuff that can be inflated with air and a pressure meter for measuring air pressure in the cuff. The pressure meter may have a pump attached configured to inflate the cuff and a button (or any other method) configured to allow the cuff to deflate. A caregiver may use methods known in the art to determine a maternal patient's systolic or diastolic blood pressure using the blood pressure cuff system. Cardiac monitor(s) 111 may further comprise optical sensors configured to measure blood oxygenation and/or pulse. These optical sensors may be affixed to the maternal patient's finger or affixed to the maternal patient's abdomen. The optical sensors are configured to conduct reflective measurement of maternal patient circulation in the finger or abdominal tissue.

Labor duration monitoring hardware 102 may include any sensor or hardware configured to sense the progress of labor. Labor duration monitoring hardware 102 may exemplarily comprise a TOCO dynamometer to noninvasively measure changes of the abdominal shape induced by uterine contractions using toco transducers. Labor duration monitoring hardware 102 may exemplarily comprise a device that uses Electrohyterography (EHG) or Electromyography (EMG) to measure the transabdominal electrical activity to measure uterine activity. Labor duration monitoring hardware 102 may exemplarily comprise an accelerometer configured to detect motion artifacts to sense if the maternal patient is moving and/or to differentiate movements associated with contractions from other patient movements. Labor duration monitoring hardware 102 may exemplarily include a microphone configured to detect the maternal patient's voice throughout the course of labor. For example, as the course of labor progresses the maternal patient's voice may become louder and may change frequency or durations of loudness, allowing the microphone and associated control system to track the course of labor.

User interface 106 facilitates the input of information, such as regarding the maternal patient's health status, the stage of labor, stimulation parameters, or other information. User interface 106 may comprise a screen and/or inputs on a wearable patient monitoring device worn by the patient, such as on a housing of the EMG/stimulation system 109, on a housing of cardiac monitors 111, or on a housing of a multi-modality bedside patient monitoring system. Alternatively or additionally, the user interface 106 may comprise a computer terminal connected to a central computing network at the healthcare facility where the maternal patient is being treated.

Control system 99 comprises central computer 101 and any computing or control elements of the various patient monitoring systems, including a controller in the ultrasound system 105 and/or a controller in the EMG/stimulation system 109. In varying exemplary embodiments, control system 99 may be any arrangement of controllers which can perform the control functions described herein. The control system 99 includes one or more hardware processing devices, or controllers, and the functions described herein may be executed by distributed processes across multiple hardware processors communicatively connected, or may be executed by a single hardware processor.

The central computer 101 comprises one or more processors configured to access a memory 103 storing one or more computer programs executable to perform the functions and methods described herein.. The computer programs may also include stored data. The computer program(s) include processor-executable instructions that are stored on a non-transitory tangible computer readable medium comprising memory 103. Non-limiting examples of the non-transitory tangible computer readable medium include nonvolatile memory, magnetic storage, and optical storage.

The central computer 101 may be communicatively connected to the patient's electronic medical record (EMR) 107 so as to receive information therefrom. The patient's EMR 107 may give central computer 101 information about the patient's uterine or other health history, which may help indicate the patient's risk of PPH. Alternatively or additionally, the central computer 101 may write information to a patient's EMR, such as a risk score indicating a likelihood and/or severity of PPH or a treatment history indicating a treatment given for PPH.

Maternal patient monitoring system 100 further comprises communication link 108, which may be a wired or wireless means of communication and is configured to communicate data between devices or subsystems. For example, communication link 108 may be a radio communication link, such as any device known in the art for wirelessly transmitting data between two points. In one embodiment, radio communication link 108 may be a body area network (BAN) device, such as a medical body area network (MBAN) device, that operate as a wireless network of wearable or portable computing devices. In such an embodiment, one or more generic activator modules which may be connected to various sensor devices attached to the patient may be in communication with a host device positioned in proximity of the patient. Other examples of radio protocols that could be used for this purpose are Bluetooth, Bluetooth Low Energy (BLE), ANT and ZigBee. Alternatively, communication link between some devices or subsystems may be a physical link, such as a cable, configured for communication of data and/or analog signals.

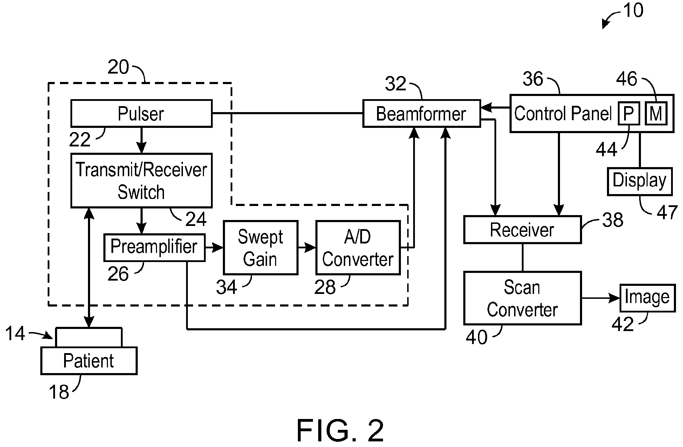

FIG. 2 depicts an ultrasound system 10 that may be employed in accordance with the present approach. The disclosed ultrasound system 10 may be employed alone, or in conjunction with other patient monitoring modalities and/or muscle stimulation systems disclosed herein. The illustrated ultrasound system 10 includes a transducer array 14 (e.g., an array of transducer elements arranged together as a single transducer) having transducer elements (e.g., piezoelectric crystals) suitable for contact with a subject or maternal patient 18 during an imaging procedure. The transducer array 14 comprising transducer elements may be made by assembling individual transducer crystals or may be a thin film array made by thin film deposition process. Each transducer element in the transducer array 14 may be individually triggerable to transmit and/or receive. The transducer array 14 may be configured as a two-way transducer capable of transmitting ultrasound waves into and receiving such energy from the subject or maternal patient 18. In such an implementation, in the transmission mode the transducer array elements convert electrical energy into ultrasound waves and transmit the ultrasound waves into the maternal patient 18. In reception mode, the transducer array elements convert the ultrasound energy received from the patient 18 (backscattered waves) into electrical signals (or scan data).

Each transducer element in the transducer array 14 is associated with respective transducer circuitry, which may be provided as one or more application specific integrated circuits (ASICs) 20, which (although depicted outside the transducer array 14) may be housed with (e.g., in the same housing as) the transducer array 14. That is, each transducer element in the transducer array 14 is electrically connected to a respective pulser 22, transmit/receive switch 24, preamplifier 26, swept gain 34, and/or analog to digital (A/D) converter 28 provided as part of or on an ASIC 20. In other implementations, this arrangement may be simplified or otherwise changed. For example, components shown in the integrated circuit 20 may be provided upstream or downstream of the depicted arrangement, however, the basic functionality depicted will typically still be provided for each transducer element. In the depicted example, the referenced circuit functions are conceptualized as being implemented on a single ASIC 20 (denoted by dashed line); however, in other embodiments, some or all of these functions may be provided on the same or different integrated circuits. The transducer circuitry may be used to control the switching of the transducer elements. The transducer circuitry may also be used to group the transducer elements into one or more sub-apertures (e.g., in response to control signals from the control system 36).

Also depicted in FIG. 2, a variety of other imaging components are provided to enable image formation with the ultrasound system 10. Specifically, the depicted example of an ultrasound system 10 also includes a beam-former 32, a control system 36, a receiver 38, and a scan converter 40 that cooperate with the transducer circuitry to produce an image or series of images 42 that may be stored and/or displayed to an operator or otherwise processed as discussed herein. The transducer array 14 may communicate the ultrasound data to the beam-former via a wired connection or wireless connection (e.g., via a wireless communication unit that is part of the transducer array that communicates over a wi-fi network, utilizing Bluetooth ^{®} technique, or some other manner). A control system 36 for the ultrasound system 10 includes at least one hardware controller having a processing component 44 (e.g., a microprocessor) and a memory component 46, may be configured to execute stored routines for processing the acquired ultrasound signals to generate meaningful images and/or motion frames of the entire uterus, which may be displayed on a display 47 of the ultrasound system 10. The control system 36 (e.g., in response to the movement of a maternal patient) may utilize one or more algorithms (e.g., stored in memory component 46) to alter where to focus the transducer elements and/or a firing sequence (timing) for triggering the transducer elements. The control system 36 may utilize a trained machine learning model stored in memory component 46, as described below, to compare the generated ultrasound images to baseline ultrasound image(s), for example images of a healthy uterus. The processing component may use the comparison to identify the presence of an indicator of PPH, such as a uterine atony, a retained placental tissue, or a presence of endometritis. When a plurality of ultrasound images has been acquired the control system 36 may then identify the presence of an indicator of PPH by comparing the plurality of uterus images and checking if there has been a change in the uterine image indicating the presence of an indicator of PPH.

Ultrasound information may be processed by other or different mode-related modules (e.g., B-mode, Color Doppler, power Doppler, M-mode, spectral Doppler anatomical M-mode, strain, strain rate, and the like) to form 2D or 3D data sets of image frames and the like. For example, one or more modules may generate B-mode, color Doppler, power Doppler, M-mode, anatomical M-mode, strain, strain rate, spectral Doppler image frames and combinations thereof, and the like. The image frames are stored and timing information indicating a time at which the image frame was acquired in memory may be recorded with each image frame. The modules may include, for example, a scan conversion module to perform scan conversion operations to convert the image frames from Polar to Cartesian coordinates. A video processor module may be provided that reads the image frames from memory and displays the image frames in real time while a procedure is being carried out on a patient. A video processor module may store the image frames in an image memory, from which the images are read and displayed. The ultrasound system 10 shown may comprise a console system, or a portable system, such as a hand-held or laptop-type system.

The ultrasound system 10 may be operable continuously or at predetermined intervals to acquire ultrasound scan data at a frame rate that is suitable for the imaging situation. Typical frame rates may range from 20-120 fps but may be lower or higher. The acquired ultrasound scan data may be displayed on the display 47 at a display-rate that can be the same as the frame rate, or slower or faster. An image buffer may be included for storing processed frames of acquired ultrasound scan data that are not scheduled to be displayed immediately. Preferably, the image buffer is of sufficient capacity to store at least several minutes worth of frames of ultrasound scan data. The frames of ultrasound scan data are stored in a manner to facilitate retrieval thereof according to its order or time of acquisition. The image buffer may be embodied as any known data storage medium. Notably, the frame rate for imaging the uterus may be relatively low since the uterus is not rapidly moving. For example, the frame rate may be one image every few seconds, or even once every few minutes.

The display 47 may be any device capable of communicating visual information to a user. For example, the display 47 may include a liquid crystal display, a light emitting diode (LED) display, and/or any suitable display or displays. The display 47 can be operable to present ultrasound images and/or any suitable information.

Components of the ultrasound system 10 may be implemented in software, hardware, firmware, and/or the like. The various components of the ultrasound system 10 may be communicatively linked. Components of the ultrasound system 10 may be implemented separately and/or integrated in various forms.

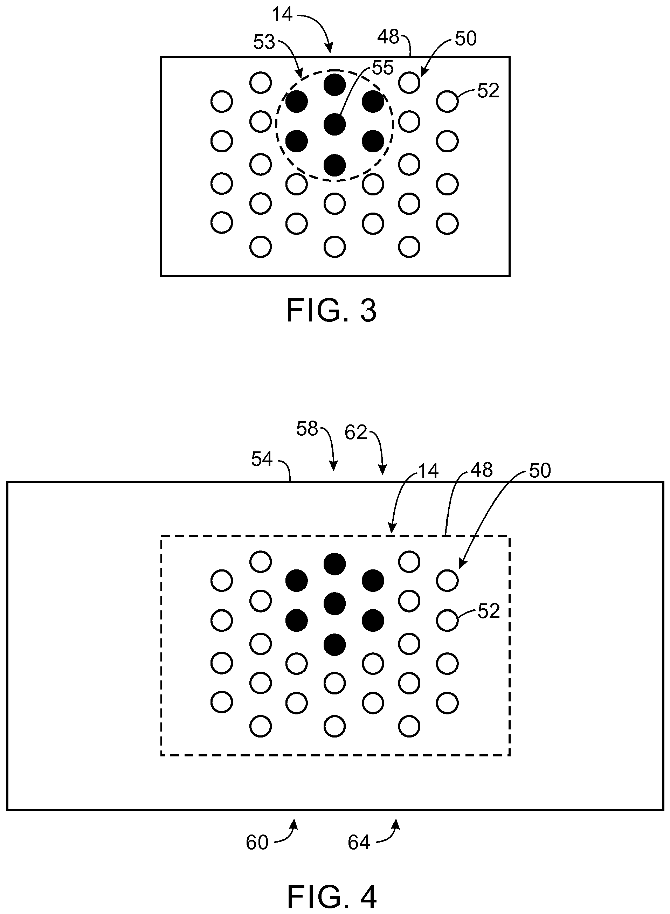

FIG.3 is a schematic diagram of one embodiment of the transducer array 14 (e.g., cooperating together to form a single ultrasound transducer system). Here, the transducer array 14 includes a flexible substrate 48 and a flexible array 50 formed of transducer elements 52 (e.g., piezoelectric crystals) on or connected together by the flexible substrate 48. The transducer may be made by assembling individual transducer crystals onto the flexible substrate 48 or may be a thin film array made by thin film deposition process. In certain embodiments, the transducer elements 52 may be made of lead zirconate titanate (PZT). In various embodiments, the flexible substrate 48 may be formed of any of various flexible, biocompatible materials, such as silicone or polymers such as polypropylene, polyamide, polycarbonate, or the like. The substrate may also be composed of a biocompatible fabric, such as polyester, and/or organic fibers such as cotton. The flexible substrate 48 may be stretchable, such as to elastically deflect over the maternal abdomen and change shape with the maternal abdomen throughout the birthing process. A variety of different materials may be suitable for such a substrate including ferro-electric polymers from PVDF family of materials.

The number of transducer elements 52 forming the flexible array 50 may vary from 10 to 100 or more transducer elements 52. During operation, the transducer elements 52 function to acquire data for imaging an entire uterus of a maternal patient and/or identify one or more indicators of postpartum hemorrhage (PPH), such as a uterine atony, retained placental tissue, or presence of endometritis. Different subsets of the transducer elements 52 (e.g., 2 or more transducer elements 52 but less than the total number of transducer elements 52 that form the flexible array 50) are triggered or fired sequentially (i.e., to transmit ultrasound waves). Thus, one subset of transducer elements 52 may be fired or triggered, then a different subset of transducer elements 52 may be fired or triggered, and the firing sequence continues until each of the transducer elements 52 have been fired or triggered. In certain embodiments, the data is acquired utilizing sub-aperture full matrix capture (FMC). In certain embodiments, the transducer elements 52 may be sequentially fired or triggered individually (as opposed to with a subset). The different subsets may have the same number of transducer elements 52 or vary in the number of transducer elements 52 making up the subset. All transducer elements 52 of the flexible array 50 receive the ultrasound energy returned from the patient. For example, assume 6 of the transducer elements 52 form a subset 53 of the transducer elements 52 and transducer element 55 of the subset 53 transmits ultrasound waves, then transducer element 55, the rest of the subset 53 of transducer elements 52 and all of the transducer elements 52 outside the subset 53 receive the ultrasound energy returned from the patient. The layout of the transducer elements 52 as depicted is two-dimensional (2D) scaled. This utilization of the transducer elements 52 ensures sufficient depth of field for Doppler measurements to enable measurement of indications of postpartum hemorrhage (PPH). As described in greater detail below, the acquisition of scan data from the sequential firing of the transducer elements enables the localization of indicators of PPH within the uterus.

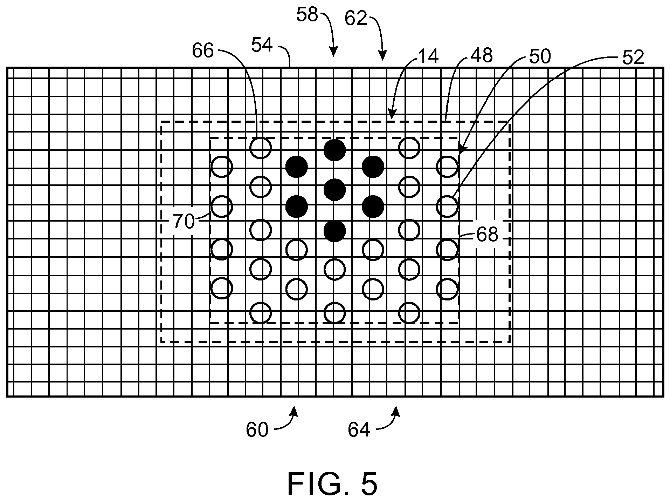

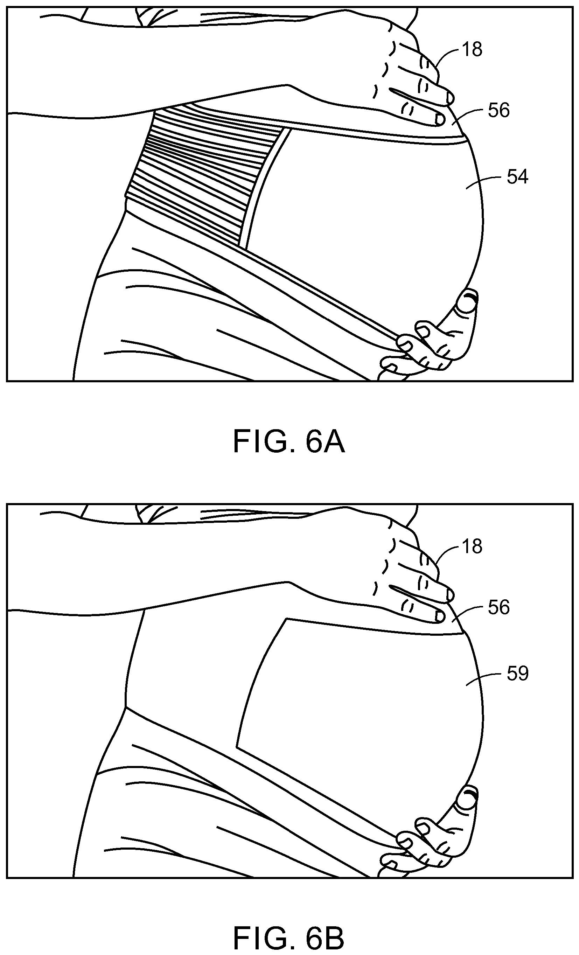

As depicted in FIG. 4, the flexible substrate 48 may be part of a single flexible belt 54 that is configured to be disposed about the abdomen of the patient (e.g., such as a flexible belt 54 in FIG. 6A disposed about an abdomen 56 of a maternal patient 18). The flexible substrate 48 may be part of the flexible belt 54 or coupled to the flexible belt 54. The transducer array 14 is disposed on an inner surface of the belt 54 and oriented so that the transducer elements 52 face the abdomen. The belt 54 may be designed to be comfortable on the patient. For example, the flexible belt 54 may be a flexible belt made of a stretchable mesh material, such as an elastic belt, as shown in FIG. 5 (e.g., similar to the material utilized with postpartum underwear), that is thin, soft, breathable, and cool. The flexible belt 54 may also be stretchy so as to conform to the different sizes of patients. In certain embodiments, the flexible belt 54 may have full openings 58, 60 on respective ends 62, 64 (e.g., superior and inferior ends) to enable the flexible belt 54 to be slid along the patient's body until it is disposed about the abdomen. In certain embodiments, the flexible belt 54 may be adjustable for fit and wrapped around the abdomen of the patient (e.g., similar to abdominal binders) and secured via fasteners (e.g., hook and loop fasteners). In other embodiments, the transducer array may be secured to other fabrics or wearable means, such as a shirt or other fitted garment.

In still other embodiments, the transducer array 14 may be attached to and maintained on the maternal abdomen by other means. For instance, as shown in FIG. 6B, the flexible substrate 48 may be part of a patch 59 adhering to the abdomen 56 of a maternal patient 18. The patch 59 is configured to continue to adhere to the abdomen 56 of the maternal patient 18 as the abdomen 56 changes shape throughout the birthing process. The patch 59 may be comprised of a flexible material upon which the flexible substrate 48 is mounted or otherwise embedded. For example, the patch 59 may comprise a flexible foam material configured to expand and contract to allow the movement of the flexible substrate 48 to conform to the changing shape of the maternal abdomen throughout the birthing process. Alternatively, the patch may be comprised of the flexible substrate material, and thus be one with the flexible substrate 48, such that the transducer elements 52 are mounted on the substrate material of the patch. The patch 59 is configured to maintain the transducer area 56 against the skin of the maternal abdomen sufficiently to enable the ultrasound imaging by the transducer array 14 of the entire uterus described herein.

The patch 59 may adhere to the skin of the maternal abdomen via a biocompatible adhesive, such as a pressure-sensitive adhesive (PSA) such as made from materials like acrylic, silicone, and rubber. The patch 59 may have adhesive on its outer edges, or on portions thereof, surrounding the transducer area. The patch 59 may have adhesive strategically placed around the transducer area 56 and configured to facilitate the flexing of flexible substrate 48 with the maternal patient's abdomen. For example, the patch 59 may have adhesive on its lateral sides, outside of the transducer area 56 and adjacent to the sides 68 and 70 (see FIG. 5). Alternatively or additionally, the patch 59 may include adhesive at strategic and intermittent locations along the top and bottom edges above and below the transducer area 56, such as centered above and below the transducer area 56 and/or above and below the lateral sides 68 and 70 of the transducer area 56 so as to maintain the transducer array against the maternal abdomen to enable continued imaging throughout and after the delivery process.

As depicted in FIG. 5 the transducer elements 52 are spread across the transducer area 66. Transducer area 66 is an area on the flexible substrate occupied by the transducer elements and is configured to image an entire uterus of a maternal patient from one location on the skin surface of maternal abdomen and without being moved. As transducer area 66 remains constant while the size of maternal uterus varies from patient to patient, the transducer area 66 is configured such that different sized uteruses may be fully imaged when the transducer array 14 is placed on the maternal patient. Thus transducer area 66 is configured to image an area within the patient that is large enough to span the patient's entire uterus, such as via beam steering, without moving or repositioning the transducer array 14 on the skin surface of the maternal abdomen. Adjusting where to focus the transducer elements 52 and/or changing the firing sequence (e.g., timing) of the transducer elements 52 enables the transducer area 66 to be held constant while the area of the uterus to be sized changes from patient to patient.

Transducer area 66 is the area on the flexible substrate occupied by the plurality of transducer elements 52 and has a first side 68 and an opposite second side 70. For example, the plurality of transducer elements may be assembled as individual transducer crystals or may be a thin film array made by a thin film deposition process. Transducer area 66 exemplarily has a width between the first side 68 and the second side 70 of about 15 cm and a height of about 10 cm, although other combinations of width and height will be apparent to those skilled in the art. In various embodiments, the transducer area may be at least 10 cm wide, such as between 10 cm and 20 cm wide, and at least 5 cm high, such as between 5 cm and 10 cm (or taller, such as a height of 15 cm). In one such embodiment, the transducer area 66 may contain an array of transducer elements 52 that is 512 elements across (distributed across the width) and 64 elements vertical (distributed across the height). In some embodiments, the transducer elements 52 are sequentially fired from the first side to the second side to image the entire uterus. The transducer area 66 may smaller than the area inside the patient that is imaged. The area imaged in the patient may have, for example, a width of about 20 cm, a height of about 30 cm, and a penetration depth of about 20cm, and the transducer array 14 is configured to emit ultrasound to penetrate the abdomen to an appropriate area and depth to image an area inside the patient that spans the entire uterus.

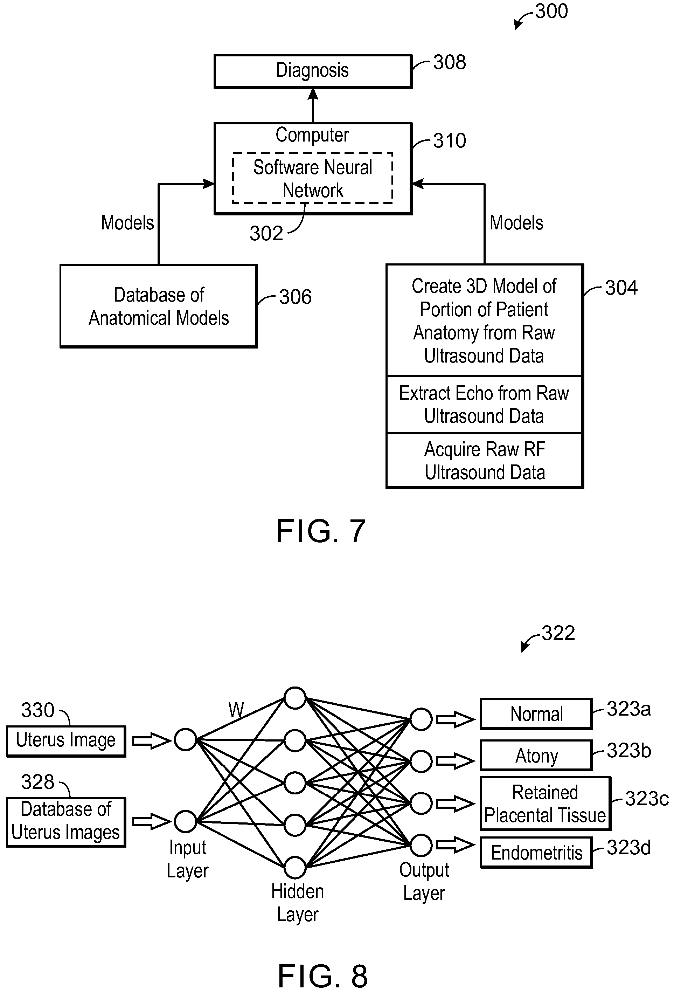

The ultrasound system 10 may be configured such that the ultrasound images are assessed and translated into data usable for assessing PPH, including identifying one or more PPH indicators. In some embodiments, the PPH indicator(s) may be identified by a doctor or other clinician assessing the ultrasound image(s) and manually entering information indicating positive or negative for various PPH factors, such as those listed herein. Alternatively, the ultrasound system 10 may be configured to automatically process the ultrasound images to identify one or more indicators of PPH. FIG. 7 illustrates one embodiment in which, the ultrasound images 42 are send to the computer 310 configured to process the images with software configured to detect indication(s) of PPH. The diagnostic system 300 may include a software based neural network 302, which may be in the form of program code residing in the memory of computer 310. The diagnostic system 300 may operate to identify an indication of PPH such as a uterine atony, an endometritis, or a retained placental tissue. A first module 304 may output a 3-D model of a portion of the patient's anatomy, specifically the uterus of a maternal patient, to the computer 310 for data processing by way of neural network 302. A second module 306 may include a database of anatomical datasets or models and may output one or more of these models to the computer 310 for processing by the neural network 302. That is, the 3-D model may be compared to the database of models by the neural network 302. The neural network 302 may then return a diagnosis based on the comparison. The data processing may provide one or more of a visual output, an audible output, and a diagnosis 308 by way of a suitable visual display, such as the display 47.

FIG. 8 illustrates one embodiment of a neural network classifier 322 having multiple outputs 323a, 323b, 323c, 323d (e.g., binary outputs where each output is either a "1" or a "0" wherein the "1" corresponds to "yes" and the "0" corresponds to "no"). In this neural network classifier, each output 323a, 323b, 323c, 323d represents the response of the neural network 302 to a particular set of inputs containing one or more indicator of PPH. For example, one output 323a may represent a normal condition where indicators of PPH are not present, while another output 323b may represent the response for the presence or absence of a uterine atony. In either case, the output state of the respective indicator will be "1" if the indicator is detected as being present and "0" otherwise. Similarly, the neural network 302 may output an appropriate state for other indicators of PPH, such as retained placental tissue 323c or endometritis 323d. The inputs to this neural network classifier are an image of the patient's uterus 330 and a database of uterus images 328 of healthy and diseased uteruses. The neural network 302 and the classifier 322 may be significantly more or less sophisticated, depending on the underlying model of the anatomical feature(s) in question (i.e., the uterus). Alternatively or additionally, other outputs may be include for other indicators of PPH, such as abnormal placentation-e.g., Placenta Accreta Spectrum (PAS).

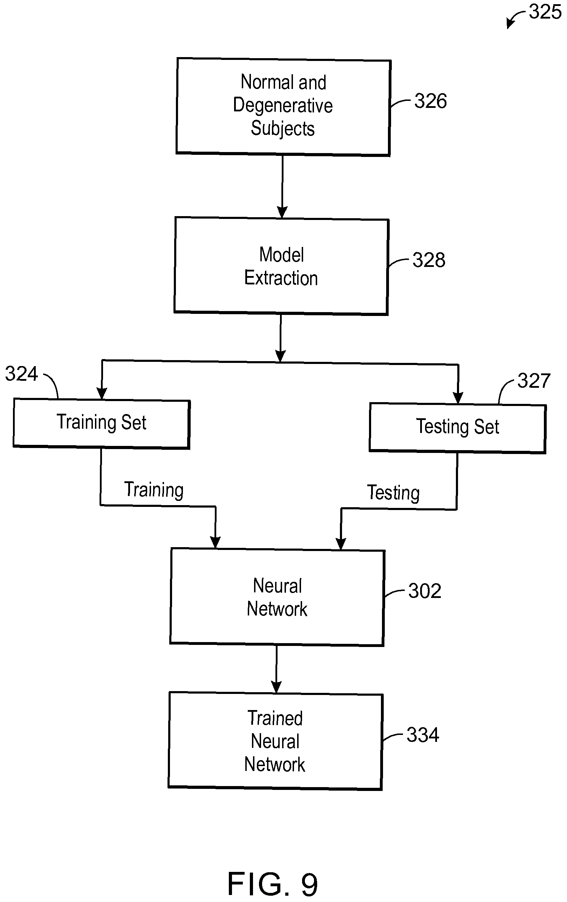

FIG. 9 illustrates one embodiment of a construction 325 of a trained neural network 334 to be utilized for detecting one or more PPH indicators based on ultrasound images acquired from a maternal patient. A group of uterus images stored in a database 328 is formulated (such by the above-described processes or by any process for generating ultrasound images of uteruses) to be used as training and testing data, which includes uterus images from subjects 326, both normal subjects and those exhibiting one or more indicators of PPH. The construction 325 includes formulating a supervised classifier using a labeled training set 324 of uterus images, including normal uterus images and abnormal uterus images containing one or more indicators of PPH. The neural network 302 is trained with the training set 324, wherein each training image consists of data (e.g., 3-D ultrasound models) collected from one or more patients or test subjects and wherein each image in the training set 324 is labeled, including labels as being positive or negative for PPH indicators and whether the patient from which the images were obtained ultimately developed PPH or not. The training set may also include pre-birth images. The neural network 302 is tested using the testing set 327, such as to generate feedback for further training the neural network 302 so as to generate the trained neural network 334.

The flexible belt 54 is designed to be worn throughout and following the birthing process, including during any or all of the antepartum, intrapartum, and postpartum phases of labor. The flexible belt 54 may be donned by the maternal patient in the early stages of labor or before labor has begun. Transducer array 14 then proceeds to image the entire uterus throughout the labor process. Post-labor the flexible belt 54 continues to be worn. The transducer array 14 continues to image the entire uterus and repositioning of the flexible belt 54 is unnecessary. Flexible substrate 48 is configured to flex and adjust its shape as the maternal abdomen changes shape throughout the phases of labor, including postpartum. Flexible substrate 48 is configured to attach to the maternal abdomen via flexible belt 54 or patch 59. Flexible substrate 48 is further configured to facilitate the movement and flexion of transducer array 14 such that transducer array 14 remains positioned and substantially stationary on the maternal abdomen sufficiently such that that it can continue to image the entire maternal uterus throughout the birth process and postpartum, even as the shape of the maternal abdomen flexes and changes.

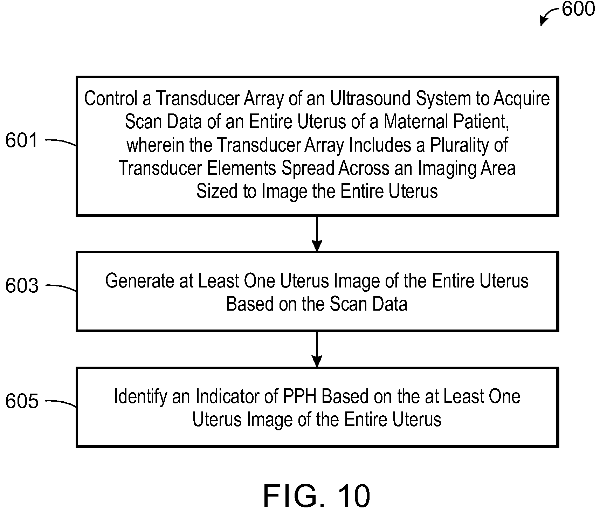

FIG. 10 is a flowchart of a method 600 for monitoring a maternal patient for PPH. At step 601 a transducer array of an ultrasound system is controlled to acquire scan data of an entire uterus of a maternal patient. The transducer array comprises a plurality of transducer elements, for example, transducer elements on a flexible substrate configured to be maintained in a stationary position on an abdomen of the maternal patient to acquire the uterus image. The plurality of transducer elements are spread across a transducer area sized to image the entire uterus of a patient without moving the transducer array. At step 603 at least one uterus image of the entire uterus is generated based on the scan data acquired in step 601. Finally, at step 605, an indicator of PPH-such as uterine atony, retained placental tissue, or presence of endometritis-is identified based on the least one uterus image of the entire uterus.

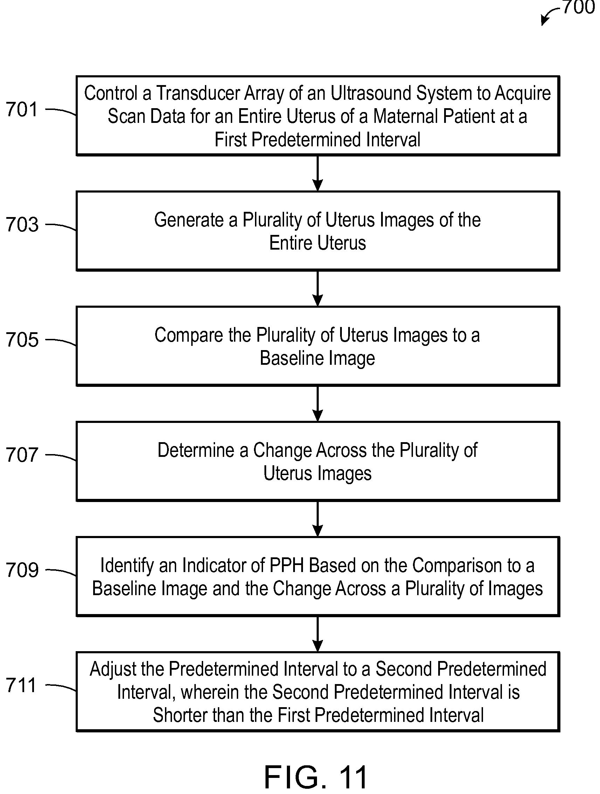

FIG. 11 is a flowchart of a method 700 for monitoring a maternal patient for PPH. At step 701 a transducer array of an ultrasound system is controlled to acquire scan data for an entire uterus of a maternal patient at a first predetermined interval. In some embodiments, controlling the transducer array further comprises activating subsets of transducer elements of the plurality of transducer elements to capture sub-aperture full matrix capture (FMC) data, generating the image of the entire uterus based on the FMC data generated by a plurality of different subsets of transducer elements. In some embodiments, the transducer array includes a plurality of subsets of transducer elements between a first side and a second side of the transducer area and controlling the transducer array further comprises activating subsets of transducer elements sequentially between the first side and the second side.

At step 703 a plurality of uterus images of the entire uterus are generated, such as ultrasound images 42 generated by the ultrasound system 10 as described above. At step 705, the plurality of uterus images are compared to a baseline image, which may be a generalized baseline depicting a healthy uterus or may be a patient-specific baseline, such as an image of the patient's uterus acquired at an earlier time. At step 707 a change is detected across the plurality of uterus images, such as between a first image and one or more later-captured images of the entire uterus. At step 709 an indicator of PPH is identified based on the comparison to a baseline image and the change across a plurality of uterus images. At step 711, the predetermined interval is adjusted to a second predetermined interval, wherein the second predetermined interval is shorter than the first predetermined interval such that the ultrasound imaging of the uterus happens more frequently.

The above-described steps of the processes of FIGS. 10 and 11 are not limited to the order and sequence shown and described in the figures and in various embodiments the steps may be performed in another order. Some of the above steps of the processes of FIGS. 10 or 11 can be executed or performed substantially simultaneously where appropriate or in parallel to reduce latency and processing times.

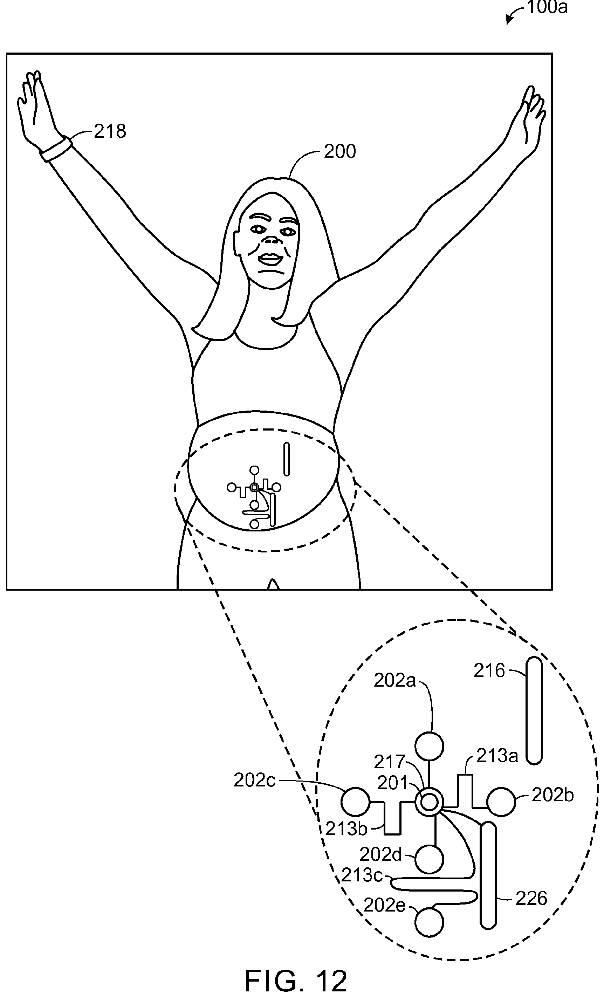

FIG. 12 illustrates one embodiment of the monitoring system 100a comprising a plurality of surface electrodes configured for EMG monitoring and stimulation and other sensors in use and attached to a maternal patient 200. The plurality of surface electrodes and other sensors may comprise a patch system such as the Novii ^{®} patch from GE Healthcare. Surface electrodes 202a-202e are disposed on the abdomen of maternal patient 200. The skin of maternal patient 200 is preferably prepared to ensure a good contact is made between each electrode and the skin, and gel is preferably applied to electrically couple the electrodes to the skin. The umbilicus 217 of maternal patient 200 may serve as a reference for applying the various surface electrodes 202a-202e and other sensors. An electronic control unit 201 may include a housing configured to physically attach to the patch or be received by an attachment mechanism, such as a holster or clip, that attaches the housing to the patch. The housing may include a port configured to electrically connect to the electrodes, and the housing may house a controller configured to receive and digitize the EMG potentials from the electrodes and process the EMG electrodes, including digital and analog filtering, to isolate the EMG signals indicating uterine activity of the maternal patient 200. The housing may also house stimulation hardware configured to generate a stimulation pulse between a subset of the electrodes 202a-202e to stimulate the uterine muscle, as is described in more detail elsewhere herein.

In an exemplary embodiment, the first sensing electrode 202b and drive electrode 202d are arranged on the abdomen on the medial plane of the subject. The common electrode 202e is placed facing the symphysis pubis, by extending the flexible substructure on which wire 213c is mounted. Surface electrodes 202a-202e may be used for EMG and/or stimulation. In some embodiments, some or all of the surface electrodes 202a-202e may be used to gather other physiological data, such as EMG signals representing the activity of the uterine activity of the maternal patient. Additionally, in some embodiments, the system may be configured to isolate philological signals indicating cardiac information of the maternal patient 200, and/or philological signals indicating cardiac information of the fetal patient.

With continued reference to FIG. 12, a blood pressure sensor 218 is shown connected to the arm of maternal patient 200. Blood pressure sensor 218 may be communicatively connected to a controller for monitoring system 100a, via a wired or wireless communicative connection. Blood pressure sensor 218 is exemplarily a blood pressure cuff as described above. Blood pressure sensor 218 may comprise a standard blood pressure cuff that can be inflated with air and a pressure meter for reading the pressure of the cuff.

An optical sensor 226 is shown disposed on the abdomen of maternal patient 200. Optical sensor 226 may alternatively be disposed on the finger of maternal patient 200. Optical sensor 226 is configured to sense the reflectivity of maternal patient 200's blood, and thereby to determine the oxygenation and/or pulse rate of maternal patient 200. Optical sensor 226 may be communicatively connected to a controller for monitoring system 100a, via a wired or wireless communicative connection.

An ultrasound sensor 216 is shown disposed on the abdomen of maternal patient 200. Ultrasound sensor 216 is configured to image all or part of the uterus of maternal patient 200. Ultrasound sensor 216 may comprise a controller which controls an ultrasound transducer to generate ultrasound image data and performs interpretation of ultrasound image data. Ultrasound sensor 216 may be in communicative connection with a controller for monitoring system 100a, via a wired or wireless communicative connection.

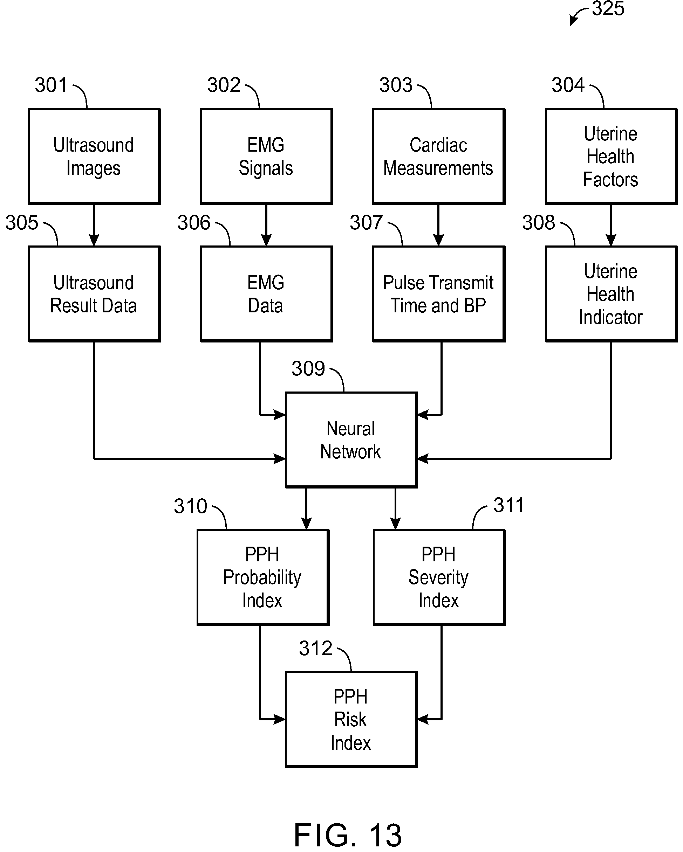

FIG. 13 shows a data system configured for generating a PPH risk index. A machine learning model is trained and used to generate a PPH probability index 310 and a PPH severity index 311 based on available inputs, such as ultrasound result data 305, EMG data 306, pulse transmit time and blood pressure 307, and uterine health indicator 308.

Ultrasound images 301 are processed into ultrasound result data 305. Ultrasound result data 305 includes information indicating the presence, absence, and/or severity of at least one condition or indicator associated with PPH, such as placental tissue remaining after birth, a uterine tear, endometritis, and/or a uterine atony. Features of the uterus are identified and extracted from ultrasound images 301. These features are analyzed to identify the presence, absence, or severity of a predetermined list of conditions or PPH indicators, such as placental tissue remaining after birth, uterine tear, endometritis, and/or uterine atony. The determination of the presence or severity of PPH indicators may be based on a single ultrasound image of a uterus acquired from the maternal patient, such as by comparing to a model or exemplary uterus image or portion thereof. The determination may alternatively be based on a plurality of uterus images, such as by tracking changes over time across a number of ultrasound images acquired from the maternal patient. In one embodiment, ultrasound images 301 of the uterus are processed within the system, such as by the central computer 101 (FIG. 1), to generate the result data. For example, an image processing machine learning model may be trained to receive and process the ultrasound images 301 from the ultrasound system and to recognize one or more indicators of PPH. Alternatively, the ultrasound images 301 may be reviewed by a clinician, who may input the ultrasound result data 305, for example via the user input device and/or into the patient's EMR such that it may be retrievable by the disclosed patient monitoring system.

EMG signals 302 are processed into EMG result data 306. EMG result data 306 may include quantifications of the EMG signals 302, such as amplitude and frequency values, and also includes one or more values indicating the presence/absence and/or severity of at least one of an abdominal muscle contraction, uterine activity, or a uterine atony. In one embodiment, EMG signals 302 are processed within the system, such as by the central computer 101 (FIG. 1), to generate the EMG result data 306. For example, a signal processing machine learning model may be trained to receive and process the EMG signals 302 from the EMG system and to recognize one or more of an abdominal muscle contraction, uterine activity, or a uterine atony. Alternatively, the EMG signals 302 may be reviewed by a clinician, who may input the EMG result data 306, for example from the user input device and/or the patient's EMR. In exemplary embodiments, EMG result data 306 is generated by comparing features of EMG signals 302 to threshold features. For example, the amplitude of EMG signals 302 is compared to a threshold amplitude. If the amplitude of EMG signals 302 is less than the threshold amplitude, and EMG result may be generated indicating the presence of an abdominal muscle contraction, uterine activity, or a uterine atony. In other exemplary embodiments, EMG result data 306 is generated by comparing EMG signals 302 to a model waveform representing the EMG response of a healthy uterus. Any deviation in EMG signal 302 from the model waveform may indicate the presence of an abdominal muscle contraction, uterine activity, or a uterine atony. In other exemplary embodiments, EMG result data 306 is generated by examining the pattern of EMG signals 302 over time. Any changes the in the pattern of EMG signals 302 may indicate insufficient uterine activity or the presence of uterine atony.

Cardiac measurements 303 are processed into pulse transmit time and blood pressure 307. Pulse transit time (PTT) is the time taken for the arterial pulse pressure wave to travel from the aortic valve to a peripheral site. Blood pressure (BP) is the amount of force your blood uses to get through your arteries and may refer to systolic blood pressure, diastolic blood pressure, or both. Cardiac measurements may further include heart rate (not shown). Cardiac measurements 303 are exemplarily taken at the extremities of a maternal patient, for example via a blood pressure cuff worn on a maternal patient's arm or an optical sensor worn on a maternal patient's finger. Alternatively or additionally, cardiac information may be obtained by electrodes on the maternal patient, which may be on the patient's chest and/or abdomen, and configured to record cardiac potentials representing aspects of the patient's heart beat.

Uterine health factors 304 are processed into uterine health indicator 308. For example, uterine health factors may be inputted by a clinician and/or obtained from the patient's medical record. Uterine health factors may include information about the maternal patient, such as uterus type, menstrual history, previous birth history, hormone health history, and the like.

The maternal patient monitoring system may be configured to generate a uterine health indicator 308 based on the uterine health factors. The uterine health indicator may be a score generated based on at least one of a uterus type, a menstrual history, a previous birth history, and a hormone health history. In one embodiment, a machine learning model is trained to generate the uterine health indicator 308 based on maternal uterine health information and used to generate the uterine health indicator 308 based on available uterine health factors 304, such as a uterus type, a menstrual history, a previous birth history, and a hormone health history. Alternatively, the uterine health factors may be reviewed by a clinician who may determine and input the uterine health indicator 308, for example via the user input device and/or the patient's EMR.

Ultrasound result data 305, EMG data 306, pulse transmit time and blood pressure 307, and the uterine health indicator 308 are inputs to a trained neural network 309. Collectively, these PPH factors are defined as PPH factor information. The trained neural network 309 is trained to calculate a PPH probability index 310 and a PPH severity index 311 based on various inputs. The neural network 309 may be, for example, a deep learning neural network trained to calculate a PPH probability index 310 and a PPH severity index 311 from the PPH factor information listed above. In various embodiments, the neural network 309 may be a machine learning model such as a classification model such as a decision tree, K-nearest neighbor, random forest, a neural network trained via supervised learning on historical and patient data, or a neural network trained via reinforcement learning. In various embodiments, the neural network 309 may be trained on PPH factor information derived from previously studied uteruses. This PPH factor information from previously studied uteruses may be stored in a database, along with information indicating whether each uterus developed PPH and, if so, what the severity of PPH was. The database of information may be split into training data and testing data. The training data may be used to train the neural network 309 to calculate a PPH probability index 310 and a PPH severity index 311 from the PPH factor information. The testing data may be used to generate feedback to continue training neural network 309.

The PPH probability index 310 represents the likelihood of PPH occurring in the maternal patient. The PPH probability index 310 may be a number or a percentage, such as between 1 and 100, representing the probability of PPH for the patient. Alternatively, the PPH probability index 310 may be one of a predetermined set of values (e.g., of high, medium, or low) indicating the probability of the patient developing PPH. The PPH severity index 311 represents a severity of PPH if PPH were to occur in the maternal patient. The PPH severity index 311 may be a number or a percentage, such as between 1 and 100, representing the severity of PPH for the patient. Alternatively, the PPH severity index 311 may be one of a predetermined set of values (e.g. of high, medium, or low) indicating the severity of PPH if the maternal patient were to develop PPH. The PPH severity index 311 may be the same or different type of value from the PPH probability index 310.

After the neural network calculates the PPH probability index 310 and PPH severity index 311, a PPH risk index 312 is determined based thereon. For example, the PPH risk index 312 may be a combination of the PPH probability index 310 and PPH severity index 311. For instance, the PPH risk index 312 may be two values, one being the PPH probability index 310 and one being the PPH severity index 311. Alternatively, the PPH risk index 312 may be a single number or indicator value combining the PPH probability index 310 and PPH risk index 311 to show the risk of PPH to a patient, such as a percentage value or a value on a different scale type, such as on a high/medium/low value scale.

It should be recognized that the system may be configured so that not all PPH factors are required or necessary to calculate a PPH risk index 312, and that the system can function to calculate the risk index 312 based on an available subset of the inputs. The system may be configured to calculate the PPH risk index 312 based on any available data, or may be configured such that some inputs are mandatory while others are optional. Similarly, the system may be configured to require a threshold amount of certain types of data, or certain input values, in order to calculate the PPH risk index. For example, the system may be configured to require a threshold duration of EMG data and/or cardiac measurements, and/or a threshold number or type of ultrasound result data inputs. In some embodiments, the system may be configured to require at least two of the PPH factors to calculate a PPH risk index 312, and in further embodiments three or more PPH factors may be required as inputs to calculate a PPH risk index 312.

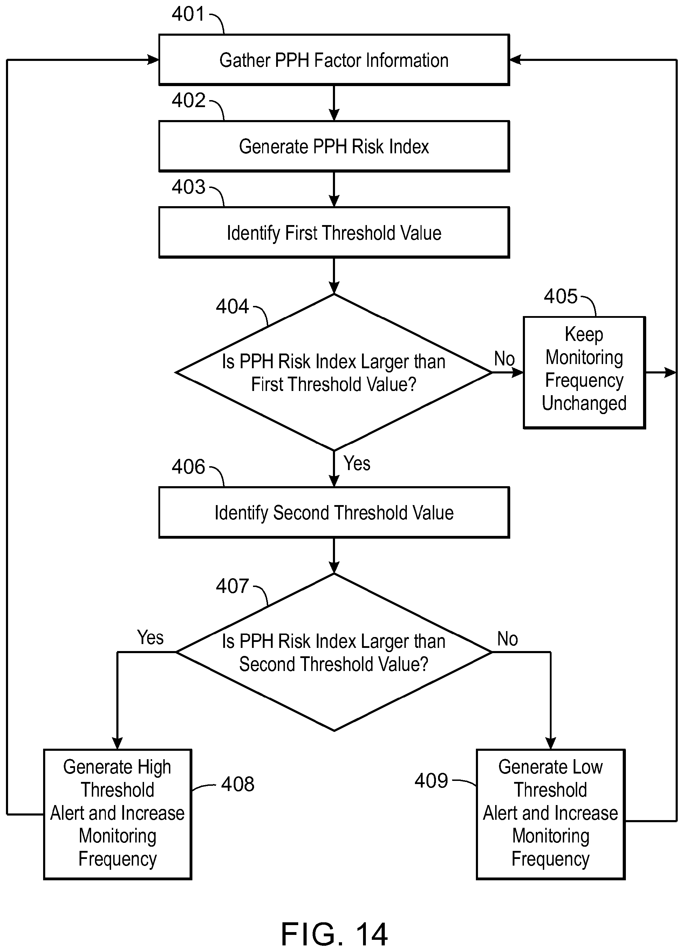

Referring to FIG. 14, a flow chart is shown describing a method for monitoring a post-partum patient and determining a risk for PPH. At step 401, PPH factor information is gathered, such as via the sensors comprising part of the monitoring system 100, 100a, via the patient's health record, and/or user input devices, etc. At step 402, a PPH risk index is generated based on the available PPH factor information. As described above, the PPH factor information may include one or more of ultrasound result data 305, EMG data 306, pulse transmit time and blood pressure 307, and the uterine health indicator 308. Calculation of the PPH risk index may include first determining a PPH probability index and a PPH severity index, as described above. The PPH risk index may be generated periodically, and the period of generating the PPH risk index may be based on a measurement period of the PPH factor information.

At step 403, a first threshold value for assessing the PPH risk index is identified, such as based on information from the patient's medical history and/or their biometric data. The first threshold value corresponds to the minimum PPH risk index value that indicates that the patient is at a high risk for PPH and monitoring should be increased. For example, the first threshold value may be selected based on a patient's age, weight, uterine health score, or other factors. The first threshold value may be automatically calculated algorithmically or selected by the system based on a patient's age, weight, uterine health score, or other factors, such as via programming executed by the central computer 101 (see FIG. 1). Alternatively, the first threshold value may be selected or otherwise inputted by a clinician, such as in response to a prompt generated on a user interface 106 (see FIG. 1). In still other embodiments, the first threshold value is a standard default value across all patients.

The calculated PPH risk index is compared to the first threshold value at step 404. If the PPH risk index is lower than the first threshold value, the monitoring frequency of PPH factor information is kept unchanged at step 405 and the process then repeats beginning with step 401. If the PPH risk index is greater than the first threshold value, a second threshold value is identified at step 406. For example, the second threshold value corresponds to a minimum PPH risk index value at which the patient should seek and/or be given immediate emergency medical attention. The second threshold value is higher than the first threshold value and/or represents a higher and/or more urgent risk to the patient's health. The second threshold value may be automatically calculated algorithmically or selected by the system, based on a patient's age, weight, uterine health score, or other factors, such as via programming executed by the central computer 101 (see FIG. 1). Alternatively, the first threshold value may be selected or otherwise inputted by a clinician, such as in response to a prompt generated on a user interface 106. In still other embodiments, the second threshold value is a standard default value across all patients.

At step 407, the PPH risk index is compared to the second threshold value. If the PPH risk index is lower than the second threshold value, that is if the PPH risk index is higher than the first threshold value but lower than the second threshold value, then frequency of monitoring is increased at step 409 and the process repeats with step 401. In some embodiments, increasing the frequency of monitoring means increasing the frequency of receiving PPH factor information and the frequency of generating the PPH risk index. Additionally, a low-threshold alert is generated at step 409 to alert the patient and/or caregiver of the risk of PPH.

If, at step 407, the PPH risk index is greater than the second threshold value, a high threshold alert is generated at step 408. The high threshold alert indicates that the patient is at a high risk of PPH and should seek and/or be given immediate emergency medical attention. The low threshold and high threshold alerts can be in the form of automated phone calls, text messages, auditory alerts, alerts which power light-emitting diodes (LEDs) of different colors, and/or any other form of alert known in the art which can alert patients and/or caregivers that a patient is at an elevated or dangerous risk for PPH. The monitoring frequency may also be increased such that the PPH factor information is gathered more frequently and the PPH risk index is calculated more frequently. In one embodiment, the monitoring frequency is increased more in response to a high threshold alert than a low threshold alert. For example, detection of a high threshold alert may trigger continuous monitoring where feasible, or otherwise monitoring at a maximum frequency enabled by the hardware and software limitations of the system 100, 100a.

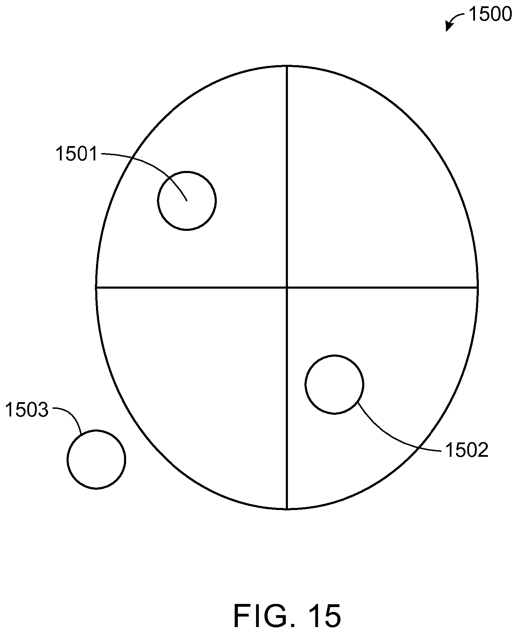

FIG. 15 shows an exemplary arrangement 1500 of a plurality of electrodes on the maternal abdomen according to an embodiment of the present invention. Electrodes 1501, 1502, and 1503 are configured to be disposed on the abdomen of a maternal patient. Electrodes 1501, 1502 and 1503 may be utilized to capture EMG signals from the maternal patient, to stimulate muscle contraction of the maternal patient, or both. Electrode 1503 acts as a reference electrode, while electrodes 1501 and 1502 act as active electrodes configured to deliver a stimulation pulse.

In some embodiments, electrode 1501 is an upper electrode positioned on the upper portion of the maternal abdomen, such as above the umbilicus or horizontal midline crossing through the umbilicus on the maternal abdomen. Electrode 1502 is a lower electrode positioned on the lower portion of the maternal abdomen, such as below the umbilicus or the horizontal midline. In other embodiments, the upper electrode 1501 may be positioned on or near the midline and the lower electrode 1502 may be low on the maternal abdomen. In still another embodiment, the upper electrode may be placed high on the maternal abdomen, such as at or near the fundus, and the lower electrode 1502 may be placed at or near the horizontal midline. In still other embodiments, both electrodes 1501 and 1502 may be located at or near the horizontal midline or arranged along the vertical midline of the maternal abdomen crossing vertically through the umbilicus. The electrodes may be located on either lateral side of the maternal abdomen, such as on either side of a vertical midline. In the depicted example, the electrodes are arranged diagonally across the maternal abdomen, with at least one electrode above the horizontal midline and to the patient-right of the vertical midline and at least a second electrode positioned below the horizontal midline and to the patient-left of the vertical midline (e.g., the positions of electrode 1501 and electrode 1502 shown in the figure). In another embodiment, the at least two electrodes may be arranged with at least one electrode above the horizontal midline and to the patient-left of the vertical midline and at least a second electrode positioned below the horizontal midline and to the patient-right of the vertical midline.

The reference electrode 1503 may be located away from the other electrodes, such as on the maternal patient's back, side, or hip area. This embodiment is merely exemplary. The arrangement 1500 of electrodes could be any arrangement of three or more electrodes, with at least a subset of the electrodes positioned on the maternal abdomen and configured to capture potentials from uterine muscle activity and/or to deliver a stimulation pulse to the maternal abdomen. Electrodes 1501, 1502, 1503 may be configured to adhere to the patient's skin. In some embodiments, electrodes 1501, 1502, and 1503 may be moveable and may be positioned at different positions on the abdomen of a maternal patient. Alternatively of additionally, the electrodes 1501, 1502, 1503 may be woven into, printed on, or otherwise integrated into a garment that is wearable by the user, such as a shirt, strap, or belt worn by the user around their abdomen.

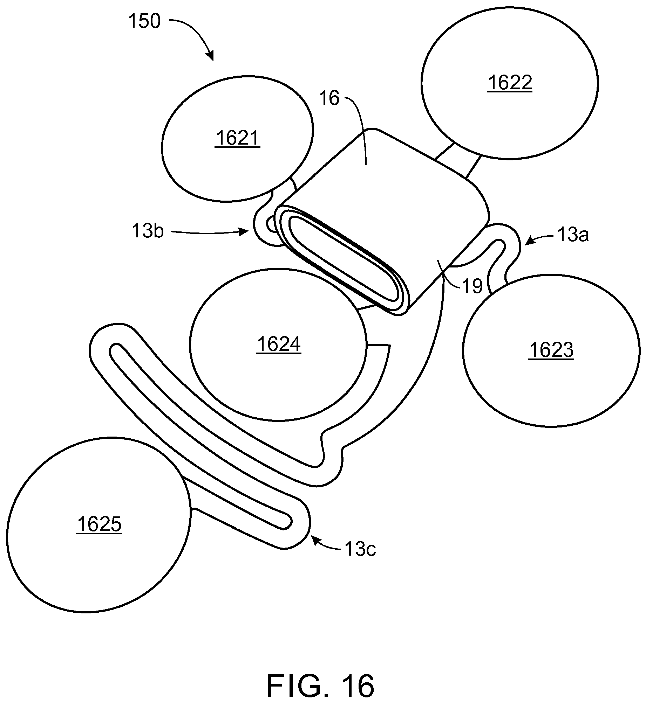

FIG. 16 shows another exemplary arrangement of electrodes integrated into a patch 150 of a plurality of electrodes according to an embodiment of the present invention. Electrodes 1621, 1622, 1623, 1624, and 1625 are skin electrodes configured to be disposed on the skin of the abdomen of a maternal patient. Electrodes 1621 - 1625 are each connected to housing 16 via wires 13a-13c. Housing 16 may include a processor 19 or other processing electronics configured to analyze the detected EMG signals and to produce physiological data of the maternal patient, including EMG signals indicating muscle activity of the uterine muscle and may also include cardiac information of the maternal patient, such as heart rate and/or ECG signals. Processor 19 and electronics may additionally be configured to control stimulation pulses delivered to the maternal patient via electrodes 1621-1625 to stimulate contraction of the uterine muscle. To that end, the processor 19 may be configured to execute one or more programs to analyze the EMG signals indicating uterine activity of the maternal patient and determine whether to stimulate and/or the magnitude of stimulation based on the EMG signals. In other embodiments, the processor 16 and electronics housed in the housing 16 may rather serve a communicative function, providing wireless communication of the detected signals from the electrodes 1621-1625 to a computer processor located remotely from the patch 150. The remotely located computer processor may perform the same functions as described herein to analyze the detected signals to produce physiological data of the maternal patient or analyze a stimulation decision to produce a stimulation control.

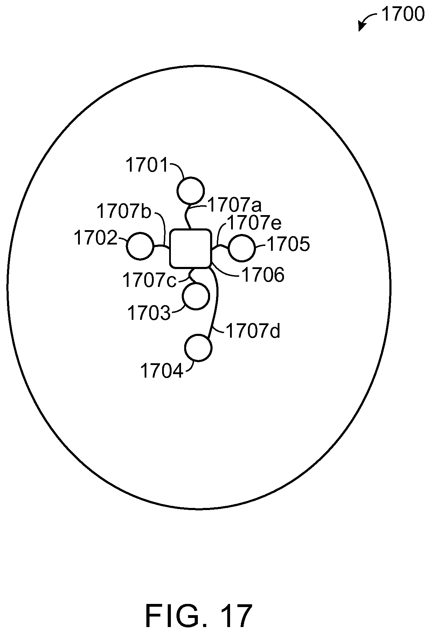

FIG. 17 shows another exemplary arrangement 1700 of electrodes. Electrodes 1701, 1702, 1703, 1704, and 1705 are configured to be disposed on the abdomen of a maternal patient. The electrodes 1701, 1702, 1703, 1704, and 1705 are connected to the control unit 1706 via wires 1707a-e. The control unit 1706 may comprise a processor and electronics configured to enable obtaining EMG signals and generating stimulation pulses and may be housed in a housing, such as housing 16 described above. In some embodiments, each of the electrodes 1701-1705 may be utilized as either a stimulation electrode or a measurement electrode. Stimulation electrodes are configured to deliver stimulation pulses to the maternal patient and may be configured in one or more pairs such that stimulation pulses are generated between a pair of two of electrodes. Measurement electrodes are configured such that the difference in electrical potential across at least two measurement electrodes is measured. In some embodiments, as first subset of the electrodes 1701-1705 is utilized as stimulation electrodes and a second subset of the electrodes 1701-1705 is utilized as measurement electrodes, where the measurement electrodes are different electrodes from those used for stimulation. Thereby, the system may be configured to record EMG signals while also delivering stimulation pulses, thus capturing the response of the uterine muscle to the stimulation pulses. In one embodiment, electrodes 1701 and 1704 are stimulation electrodes, while electrodes 1702 and 1703 are measurement electrodes, wherein electrode 1705 is utilized as a reference electrode. In another embodiment, electrodes 1702 and 1703 are the stimulation electrodes, electrodes 1701 and 1704 are measurement electrodes, and electrode 1705 is the reference electrode. In still other embodiments, electrodes 1701 and 1705 are the stimulation electrodes, electrodes 1702 and 1703 are the measurement electrodes, and electrode 1704 is the reference electrode. These embodiments are merely exemplary; any combination of stimulation electrodes, measurement electrodes, and reference electrodes is contemplated and within the scope of the present disclosure, including combinations in which some or all of the electrodes are alternately used as both stimulation and measurement electrodes.

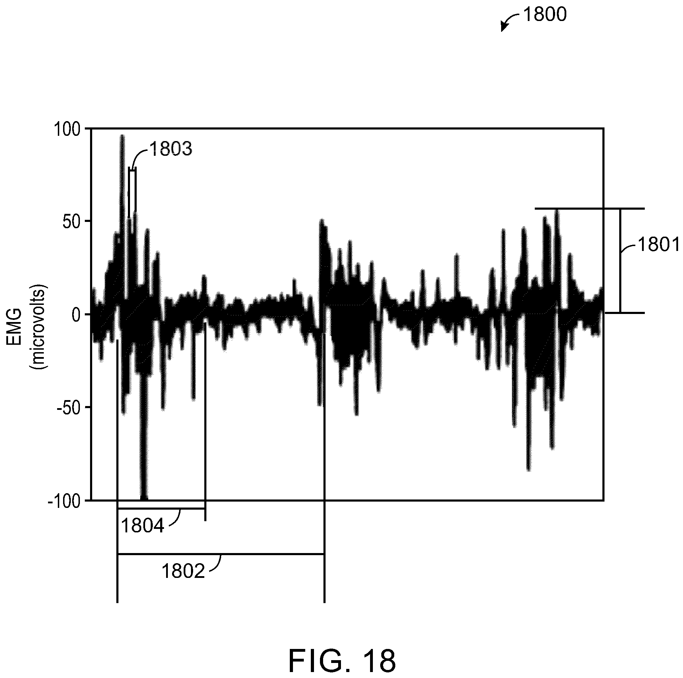

FIG. 18 shows an exemplary EMG signal 1800, generated during uterine contractions, which may be generated based on recorded potentials from a plurality of EMG electrodes used for measurement, as is variously described above. EMG signal 1800 has various features which may indicate uterine activity and may be used to determine whether or not the uterine activity of a maternal patient is sufficient at a given stage of labor, including antepartum, intrapartum, and postpartum. These features may include amplitude, frequency, contraction frequency, and contraction duration. The features may also include contraction rise time, rate of rise, etc. Referencing FIG. 18, the amplitude values derived from the EMG signal may include a peak amplitude 1801 for a contraction, average peak amplitude over a contraction period, amplitude pattern during a contraction, and/or may include peak amplitudes of every EMG peak. The frequency information derived from the EMG signal 1800 may include the frequency 1803 of the EMG signal 1800 (such as the average peak-to-peak frequency), the contraction frequency 1802, and the contraction duration 1804. The contraction duration 1804 may be measured as the duration between the start of a contraction and the end of a contraction, where the start and end of the contraction are determined based on the peak amplitudes of the EMG signal 1800. The contraction frequency 1802 may be determined using the time between the start of one contraction and the start of a subsequent contraction.

The maternal patient monitoring system 100, and specifically the control system 99 thereof, may be configured to assess the EMG signal 1800 acquired from a maternal patient to determine whether the uterine activity is sufficient. The sufficiency of uterine activity may be determined based on the frequency of the EMG signal 1803, the amplitude 1801 (e.g., peak amplitude 1801 during one or a series of contractions), the contraction duration 1804, and/or the contraction frequency 1802. The sufficiency of uterine activity may also be based on other standard ways of quantifying contractions such as Montevideo units, Alexandria units, Active Planimeter units, Total Planimeter units, Average rate of rise, etc. In one embodiment, the maternal patient monitoring system 100 may be configured to determine at least one indicator of insufficient uterine activity based on a comparison of the frequency and/or amplitude of the EMG signals to one or more frequency and/or amplitude thresholds associated with uterus health, a tired uterus condition, a presence of endometrial tissue, and/or an invasive placentation. An invasive placentation is a placenta accreta, placenta increta, or placenta percreta. Alternatively or additionally, the sufficiency of uterine activity may be based on other ways of quantifying contractions, such as Montevideo units, Total Planimeter units, Average rate of rise, etc.

In one embodiment, the frequency 1802 of the EMG signal 1800 is compared to a series of frequency thresholds or frequency patterns to determine whether and to what extent certain conditions are indicated. For example, a set of frequency thresholds associated with invasive placentation may include 20 Hz, 400 Hz, 1300 Hz, or 2000 Hz, where an EMG signal frequency of 20 Hz is associated with a normal uterus, an EMG signal frequency of 400 Hz is associated with a placenta accreta, an EMG signal frequency of 1300 Hz is associated with a placenta increta, and an EMG signal frequency of 2000 Hz is associated with a placenta percreta. As a further example, a set of amplitude thresholds associated with invasive placentation may include 1 mV, 3 mV, 6 mV, or 10 mV, where an EMG amplitude of 10 mV is associated with a normal uterus, an EMG amplitude of 6 mV is associated with a placenta accreta, an EMG amplitude of 3 mV is associated with a placenta increta, and an EMG amplitude of 1 mV is associated with a placenta percreta. These thresholds are merely exemplary; other thresholds or a combination of thresholds or a combination of values to create thresholds may be used while being within the scope of the present disclosure.

Maternal patient monitoring system 100, and specifically the control system 99 thereof, may be further configured to determine at least one indicator of insufficient uterine activity based on whether the maternal patient is the antepartum, intrapartum, or postpartum phase of labor. The system may be configured to utilize different thresholds for each stage of labor, such that during the antepartum phase an indicator of insufficient uterine activity includes the EMG frequency and/or amplitude being less than antepartum thresholds; during the intrapartum phase the indicator of insufficient uterine activity includes the EMG frequency and/or amplitude being less than intrapartum thresholds; and during the postpartum phase the indicator of insufficient uterine activity includes the EMG frequency and/or amplitude being less than postpartum thresholds.

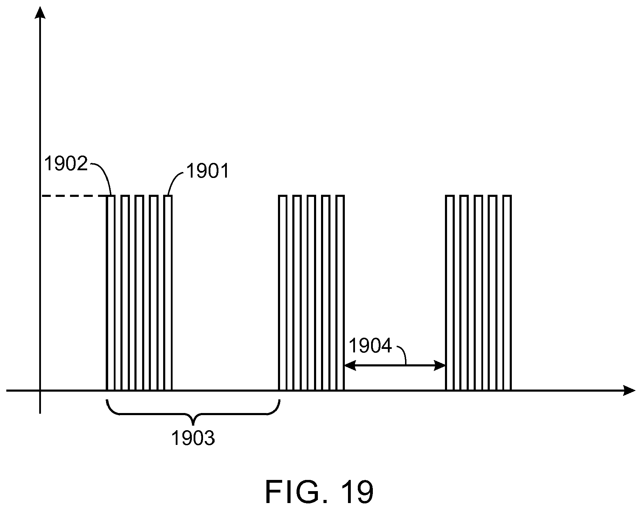

The maternal patient monitoring system 100, and specifically the control system 99 thereof, may be configured to control stimulation pulses delivered through a pair of electrodes to stimulate the uterine muscles of the maternal patient, such as via the electrode arrangements variously described above. FIG. 19 shows an exemplary embodiment of stimulation pulses and characteristics according to the present disclosure. Various parameters of the stimulation pulses may be controlled, including pulse duration, pulse width, number of pulses, stimulation frequency, and stimulation amplitude. These parameters may be adjusted to tailor stimulation delivery to the maternal patient, such as based on the stage of labor (e.g., antepartum, intrapartum, postpartum), the purpose and goal of the stimulation, and/or feedback provided by EMG signals measured from the maternal patient. The pulse duration 1901 is the duration of continual stimulation, which in the depicted example is 250 µs. The stimulation amplitude 1902 is the magnitude of the stimulation, which in the depicted example is 250 mA. The stimulation frequency 1903 is the frequency that the pulses are delivered, which in the depicted example is 20 Hz.

Another stimulation parameter may include the number of pulses delivered in quick succession, forming a stimulation session. For each stimulation session, the number of pulses may be anywhere from 1 to 10 or more. In the depicted example, the number of pulses is 5, with a 50% duty cycle and with an on-time (pulse duration) of 250 µs.

The number of pulses per stimulation session, in conjunction with the stimulation amplitude, pulse duration, and stimulation frequency, will affect how much the uterine muscle is stimulated. If the goal is to incite labor contractions, the stimulation parameters may be tuned to generate uterine muscle contraction strength, duration, and frequency that is desired for the stage of labor that the maternal patient is in. If the stimulation is being provided after delivery (i.e., after the fetus has exited the uterus) the goal of stimulation may be to incite the uterus to shrink. Hard stimulation, such as a large number of pulses per stimulation session, may result in faster uterine shrinkage. Soft stimulation, such as a lesser number of pulses per stimulation session, may result in slower uterine shrinkage.

The number of pulses and number of pulse sessions may be operator-configurable (e.g., by a clinician or by the maternal patient), or the control system 99 may be configured to automatically adjust these values based on at least one indicator of insufficient uterine activity. In some embodiments, the maternal patient monitoring system 100 is configured to obtain EMG signals between stimulation sessions to measure and monitor the uterine muscle response to the stimulation. The time between stimulation pulses represents the response read time 1904. After a set of stimulation pulses is delivered, stimulation pulses are paused to measure the response of the uterine muscle. EMG signals may be obtained during the response read time 1904, which may be from different electrodes than utilized to deliver the stimulation, or may be the same electrodes. Various exemplary electrode arrangements for stimulation and EMG measurement are described above. Response read time 1904 is exemplarily 1.5 seconds, but in other implementations may be longer or shorter. It should be understood that these values are merely exemplary and the pulse width 1901, amplitude 1902, frequency of stimulation 1903, response read time 1904, etc. illustrated in FIG. 19 could be different values while remaining within the scope of the present disclosure.

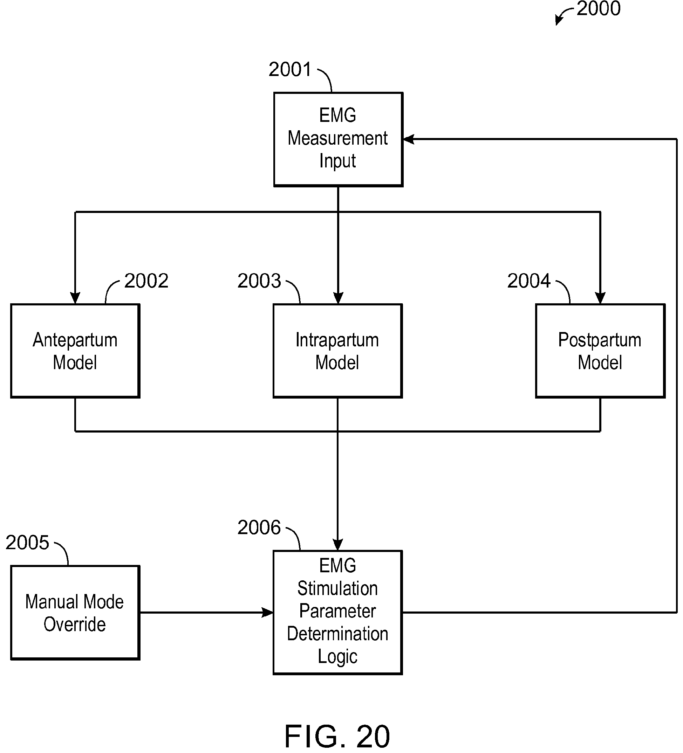

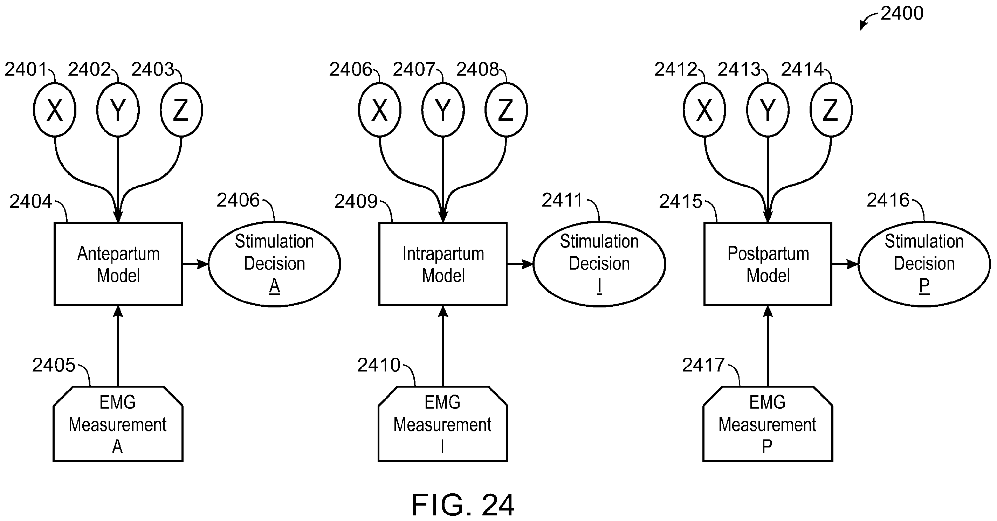

FIG. 20 shows a data structure diagram 2000 illustrating one embodiment of a system for determining and controlling stimulation. At step 2001 EMG measurements are provided, such as EMG data (recorded EMG signals and/or EMG result data) gathered by the EMG system 109 described above. The EMG data is provided as input to one of the antepartum model 2002, the intrapartum model 2003, or the postpartum model 2004, respectively. The antepartum model 2002 is a trained machine learning model stored in memory and trained to detect insufficient uterine activity based on EMG signals obtained during the antepartum phase of labor. The intrapartum model 2003 is a trained machine learning model stored in a memory and trained to detect insufficient uterine activity based on EMG signals obtained during the intrapartum phase of labor. The postpartum model 2004 is a trained machine learning model stored in memory and trained to detect insufficient uterine activity based on EMG signals obtained during postpartum phase of labor. Exemplary embodiments of such models is described in more detail with respect to FIG. 24.

One of the models is selected and utilized based on the phase of labor that the maternal patient is in. Which model is used may be selected based on clinician or other user input indicating the phase of labor, or the system may include software configured to determine the phase of labor based on physiological information gathered by sensors.

Each model is configured to output a decision on whether stimulation is to be provided, and then stimulation parameter determination logic 2006 is executed to determine stimulation parameters, such as based on the stage of labor, the EMG data, the stimulation goal to be achieved during that stage, uterine health information, previous stimulation parameters of previous stimulation pulses administered to the maternal patient, etc. EMG stimulation output is then commanded based on the stimulation parameter(s) determined at by the stimulation parameter determination logic 2006, e.g., communicated to EMG stimulation hardware, to stimulate a pulse through electrodes of a certain pulse duration, amplitude, frequency, and number of pulses. In certain embodiments, the system may be configured to facilitate user input of manual mode override commands 2005, allowing a user (for example, a patient, clinician, or other caregiver) to manually override the model to select the EMG stimulation parameter or to select an increase or decrease in on or more stimulation parameters. The models 2002, 2003, 2004 may be utilized to periodically analyze the EMG data to determine whether stimulation should be continued and/or whether the target response has been achieved with stimulation and thus the stimulation can be discontinued.

The stimulation parameter determination logic 2006 may be configured as a closed loop controller configured to utilize EMG data representing the uterine response as feedback for controlling the stimulation parameters. The muscle response to the EMG stimulation is determined based on the EMG signals, such as recorded during the response read time 1904 described above. For example, the stimulation parameter determination logic 2006 may be configured to determine sufficiency of the uterine response to the stimulation, such as based on one or more frequency and/or amplitude thresholds described above. For example, the frequency and/or amplitude thresholds utilized may be based on the stage of labor and/or other factors, as described above. If the uterine response to the stimulation was satisfactory, the stimulation parameter determination logic 2006 may maintain the same stimulation parameters between stimulation sessions. If the uterine response does not meet one or more thresholds or other criteria, stimulation parameter determination logic 2006 may be configured to increase one or more of the stimulation parameters for the next stimulation session, such as to increase the frequency, pulse width, pulse amplitude, and/or number of pulses if the maternal patient response to the stimulation does not meet the criteria.

In another embodiment if the uterine response does not meet one or more thresholds or other criteria despite stimulation, the system may generate an alert to alert a clinician and/or patient to a lack of uterine response. This may provide information to a clinician, based on which the clinician may make some determinations about the uterine conditions which can lead to decisions of for example, a C-section delivery or preparedness for, for example, PPH. For example, if the stimulation response of the uterus is insufficient, such as below a minimum threshold based on the EMG data, that may be an indicator of a serious problem with the patient's uterus (such as a tired uterus that cannot function properly). Thus, the system may be configured to generate an alert of alert of insufficient response if the stimulation response is determined to be too small, such as below a minimum threshold indicating that the uterus is capable of responding to the stimulation pulses.

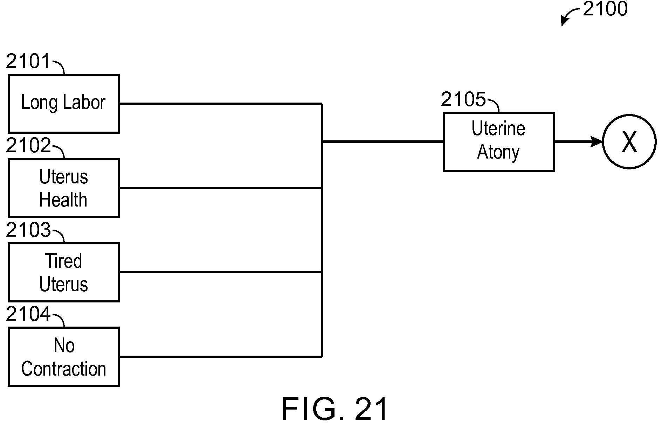

The maternal patient monitoring system 100 is designed to evaluate EMG measurements and other measurements to determine a patient's risk for PPH. Exemplary risk factors for PPH include uterine atony, placental anomaly, and adenomyosis. For each risk factor, a score may be generated representing the risk and/or severity of the condition in a maternal patient. These scores are then used to evaluate a patient's risk for PPH and as inputs for one or more computer programs used to determine whether to generate stimulation pulses to stimulate the uterine muscle of the maternal patient, as discussed below. FIG. 21 shows a data system 2100 for generating a uterine atony score 2105. The uterine atony score 2105 represents, depending on which stage of labor the maternal patient is in, the risk or severity of a uterine atony. For example, the uterine atony score 2105 may be a number from 0 to 9. Uterine atony refers to the inadequate contraction of the corpus uteri myometrial cells in response to endogenous oxytocin release. Uterine atony is a risk factor for PPH.

To evaluate the risk or severity of uterine atony, the data system evaluates a number of risk factors for uterine atony. These risk factors include long labor 2101, uterus health 2102, tired uterus 2103, and no contraction 2104. The evaluation of each factor may be based on, for example, EMG data based on EMG signals collected by the EMG system 109, ultrasound data based on ultrasound images collected by the ultrasound system 105, cardiac data based on cardiac information collected by one or more cardiac monitors 111, and/or labor duration data based on information collected by labor monitoring hardware 102. No contraction 2104 means the system has not detected the uterus contracting in the normal way after birth, which may be based on EMG data (as described in more detail below) and/or ultrasound data (as described in more detail above). For example, the presence of a tired uterus 2103 may indicate a higher risk for uterine atony, and in turn a higher risk for PPH.

Uterine atony score 2105 may be used as input to a trained neural network (e.g., one of the antepartum model 2404, the intrapartum model 2409, or the postpartum model 2415 as shown in FIG. 24) configured to generate a stimulation decision based on the input, as described in more detail below. For example, uterine atony score 2105 may be input to the antepartum model 2404, an intrapartum model 2409, or a postpartum model 2415, depending on which stage of labor the maternal patient is in. Depending on which stage of labor the patient is in, uterine atony score 2105 may or may not reflect an analysis of all uterine atony factors 2101-2104. For example, in the antepartum phase of labor the expected contraction may not have yet taken place, so the uterine atony score 2105 will not reflect an analysis of that factor.

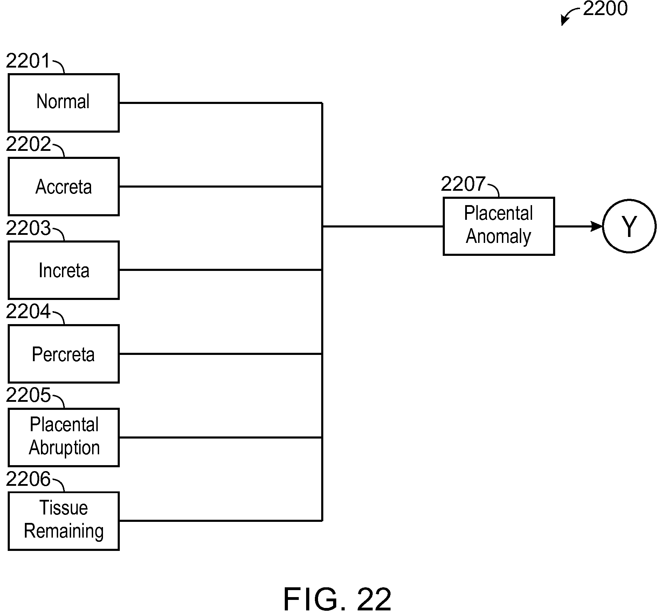

FIG. 22 shows a data system 2200 for generating a placental anomaly score 2207. Placental anomaly score 2207 may be used as input to a trained neural network configured to generate a stimulation decision, as described in more detail below. Depending on the labor stage in which it is determined, the placental anomaly score 2207 represents a risk and/or severity of a placental anomaly. Placental anomaly score 2207 may be, for example, a number from 0 to 9. Placental anomaly score considers Placenta accreta spectrum disorders when the placenta separates incompletely from the inner wall of the uterus after birth, Placental abruption and retained products of conception (tissue remaining) post-delivery. Placental anomaly is a risk factor for PPH. To evaluate the risk or severity of a placental anomaly, the data system evaluates a number of factors. These factors include whether there has been a tissue tear 2205, if there has been a tissue tear, how much tissue is remaining 2206, as well as normal 2201 (meaning no invasive placentation), accreta 2202, increta 2203, or percreta 2204. The evaluation of each factor may be based on, for example, EMG data based on EMG signals collected by the EMG system 109, ultrasound data based on ultrasound images collected by the ultrasound system 105, cardiac data based on cardiac information collected by one or more cardiac monitors 111, and/or labor duration data based on information collected by labor monitoring hardware 102. Invasive placentation is a condition in which the placenta embeds too deeply or even grows through the uterine wall. Accreta 2202, increta 2203, and percreta 2204, represent increasing levels of severity of an invasive placentation, which may be based on EMG data (as described above) and/or ultrasound data (also described above). Placental anomaly score 2207 may used as an input to a computer program configured to determine whether to deliver stimulation pulses to stimulate the patient's uterine muscle, such as a trained neural network (e.g., one of the antepartum model 2404, the intrapartum model 2409, or the postpartum model 2415 selected based on which stage of labor the maternal patient is in, as shown in FIG. 24). Depending on what stage of labor the patient is in, placental anomaly score 2207 may or may not reflect an analysis of all placental anomaly factors. For example, in the antepartum stage of labor the placenta would not have torn yet so the model would only evaluate the severity of accreta, increta, or percreta.

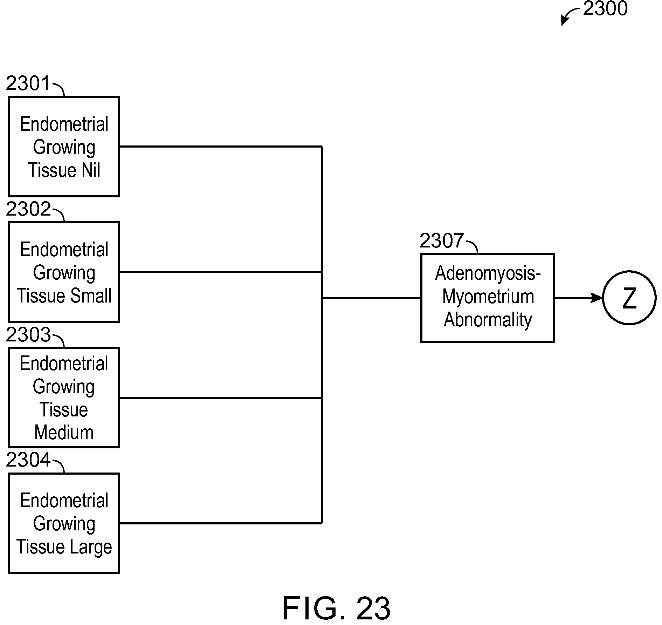

FIG. 23 shows a data system 2300 for generating an adenomyosis score 2307. Adenomyosis score 2307 represents a risk, presence, and/or severity of adenomyosis. Adenomyosis occurs when the tissue that normally lines the uterus (endometrial tissue) grows into the muscular wall of the uterus. It is a gynecological condition which can be detected pre-birth, and which serves as a risk factor for PPH. Adenomyosis score 2307 may be, for example, a number from 0 to 9. Adenomyosis score 2307 is calculated by evaluating the presence or absence of various factors. Factors include endometrial growing tissue on a scale from nil 2301 to small 2302, medium 2303, and finally large 2304. The evaluation of each factor may be based on, for example, EMG data based on EMG signals collected by the EMG system 109, ultrasound data based on ultrasound images collected by the ultrasound system 105, cardiac data based on cardiac information collected by one or more cardiac monitors 111, and/or labor duration data based on information collected by labor monitoring hardware 102. Depending on the stage of labor of the patient, adenomyosis score 2307 may or may not reflect an analysis of all adenomyosis factors. For example, the maternal patient is in the antepartum stage of labor, there will not be a tissue tear or a tissue remaining to evaluate, so the adenomyosis score 2307 would be based on the presence or absence of and severity of endometrial growing tissue.

FIG. 24 shows a data structure diagram 2400 configured to determine whether to deliver stimulation pulses to the maternal patient to stimulate uterine muscle contraction. A stimulation decision is an output indicating whether or not to deliver stimulation pulses to a patient. In some embodiments, the stimulation decision may also include one or more stimulation parameters for the stimulation. Stimulation hardware is then controlled accordingly to output stimulation to EMG electrodes disposed on the abdomen of a maternal patient.

The data structure diagram 2400 includes an antepartum model 2404, which is a trained machine learning model configured to be utilized during the antepartum phase of labor to generate a stimulation decision 2406 based on inputs, including the antepartum uterine atony, placental anomaly, and adenomyosis scores 2401, 2402, and 2403 (e.g., derived as described above), or other factors, and the antepartum EMG measurements 2405. The antepartum model is trained to evaluate these inputs and to output a stimulation decision 2406 regarding whether to deliver stimulation pulses to a maternal patient during the antepartum phase of labor. For example, stimulation pulses may be delivered to the maternal patient during the antepartum phase to stimulate labor contractions. The stimulation pulses may be delivered in response to a determination that the maternal patient is experiencing insufficient uterine activity, including insufficient labor contractions. Insufficient labor contractions may be, for example, contractions of insufficient strength (e.g., peak amplitude of the EMG signals during contractions is less than a threshold) and/or may include insufficient contraction frequency (e.g., labor contractions and/or duration are less than a threshold).

The antepartum model 2404 may be a neural network trained on antepartum uterine atony, placental anomaly, and adenomyosis scores, and antepartum EMG measurements, of historical or model patients. The antepartum model 2404 may be a deep learning neural network trained to calculate stimulation decisions based on information regarding one or more PPH indicators, such as the uterine atony, placental anomaly, adenomyosis scores, and antepartum EMG measurements. In various embodiments, the antepartum model 2404 may be a machine learning model such as a classification model such as a decision tree, K-nearest neighbor, random forest, a neural network trained via supervised learning on historical and patient data, or a neural network trained via reinforcement learning. In various embodiments, the antepartum model 2404 may be trained on antepartum uterine atony, placental anomaly, adenomyosis scores, and antepartum EMG measurements derived from previously studied or model uteruses. These scores and measurements from previously studied or model uteruses may be stored in a database, along with information indicating the ideal stimulation decision for each previously studied or model uterus. The database of information may be split into training data and testing data. The training data may be used to train the antepartum model 2404 to calculate a stimulation decision. The testing data may be used to generate feedback to continue training antepartum model 2404. In other embodiments, antepartum model 2404 is an algorithm configured to take as inputs the antepartum placental anomaly, and adenomyosis scores, and antepartum EMG measurements and to calculate or otherwise determine a stimulation decision 2406.

The data structure diagram 2400 includes an intrapartum model 2409, which is a trained machine learning model configured to be utilized during the intrapartum phase of labor to generate a stimulation decision 2411 based on inputs, including intrapartum uterine atony, placental anomaly, and adenomyosis scores 2406, 2407, and 2408 (e.g., derived as described above) and intrapartum EMG measurements 2410. The intrapartum model is trained to evaluate these inputs and to output a stimulation decision 2411 regarding whether to deliver stimulation pulses to a maternal patient during the intrapartum phase of labor. For example, stimulation pulses may be delivered to the maternal patient during the intrapartum phase to stimulate labor contractions. The stimulation pulses may be delivered in response to a determination that the maternal patient is experiencing insufficient uterine activity, including insufficient labor contractions. Insufficient labor contractions may be, for example, contractions of insufficient strength (e.g., peak amplitude of the EMG signals during contractions is less than a threshold) and/or may include insufficient contraction frequency (e.g., labor contractions and/or duration are less than a threshold). Alternatively or additionally, the sufficiency of uterine activity may be based on other ways of quantifying contractions, such as Montevideo units, Total Planimeter units, Average rate of rise, etc.

The intrapartum model 2409 may be a neural network trained on intrapartum uterine atony, placental anomaly, adenomyosis scores, and intrapartum EMG measurements, of historical or model patients. The intrapartum model 2409 may be a deep learning neural network trained to calculate stimulation decisions from the intrapartum uterine atony, placental anomaly, and adenomyosis scores and intrapartum EMG measurements. In various embodiments, the intrapartum model 2409 may be a machine learning model such as a classification model such as a decision tree, K-nearest neighbor, random forest, a neural network trained via supervised learning on historical and patient data, or a neural network trained via reinforcement learning. In various embodiments, the intrapartum model 2409 may be trained on intrapartum uterine atony, placental anomaly, and adenomyosis scores, and intrapartum EMG measurements derived from previously studied or model uteruses. These scores and measurements from previously studied or model uteruses may be stored in a database, along with information indicating the ideal stimulation decision for each previously studied or model uterus. The database of information may be split into training data and testing data. The training data may be used to train the intrapartum model 2409 to calculate a stimulation decision. The testing data may be used to generate feedback to continue training intrapartum model 2409.

The data structure diagram 2400 includes a postpartum model 2415, which is a trained machine learning model configured to be utilized during the postpartum phase of labor to generate a stimulation decision 2416 based on inputs, including postpartum uterine atony, placental anomaly, and adenomyosis scores 2412, 2413, 2414 (e.g., derived as described above) and postpartum EMG measurements 2417. The postpartum model is trained to evaluate these inputs and to output a stimulation decision 2416 regarding whether to deliver stimulation pulses to a maternal patient during the postpartum phase of labor. For example, stimulation pulses may be delivered to the maternal patient during the postpartum phase to stimulate post-labor contractions to cause uterine shrinkage. The stimulation pulses may be delivered in response to a determination that the maternal patient is experiencing insufficient uterine activity, including insufficient post-labor contractions. Insufficient post-labor contractions may be, for example, contractions of insufficient strength (e.g., peak amplitude of the EMG signals during contractions is less than a threshold) and/or may include insufficient contraction frequency (e.g., labor contractions and/or duration are less than a threshold).

Stimulation decision 2416 is a postpartum decision output by postpartum model 2415. Postpartum model 2415 takes as inputs the postpartum uterine atony, placental anomaly, and adenomyosis scores 2412, 2413, 2414 and postpartum EMG measurements 2417. Postpartum model 2415 evaluates these inputs and outputs a stimulation decision 2416. The postpartum model may be a neural network trained on postpartum uterine atony, placental anomaly, and adenomyosis scores, and postpartum EMG measurements, of historical or model patients. The postpartum model 2416 may be a deep learning neural network trained to calculate stimulation decisions from the postpartum uterine atony, placental anomaly, and adenomyosis scores and postpartum EMG measurements. In various embodiments, the postpartum model 2416 may be a machine learning model such as a classification model such as a decision tree, K-nearest neighbor, random forest, a neural network trained via supervised learning on historical and patient data, or a neural network trained via reinforcement learning. In various embodiments, the postpartum model 2416 may be trained on postpartum uterine atony, placental anomaly, and adenomyosis scores, and postpartum EMG measurements derived from previously studied or model uteruses. These scores and measurements from previously studied or model uteruses may be stored in a database, along with information indicating the ideal stimulation decision for each previously studied or model uterus. The database of information may be split into training data and testing data. The training data may be used to train the postpartum model 2416 to calculate a stimulation decision. The testing data may be used to generate feedback to continue training postpartum model 2416.

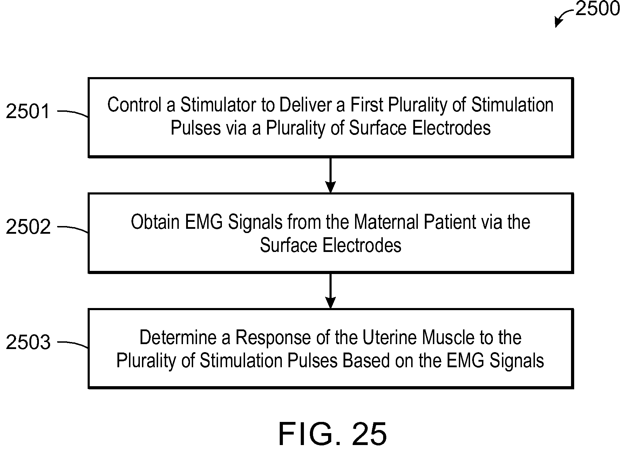

FIG. 25 shows a flow chart embodying an exemplary method 2500 for controlling uterine muscle stimulation of a maternal patient according to the present disclosure. At step 2501, a stimulator is controlled to deliver a first plurality of stimulation pulses via a plurality of surface electrodes configured to be disposed on the abdomen of a maternal patient. The first plurality of stimulation pulses are configured to stimulate a uterine muscle of the maternal patient and may be delivered by a first set of surface electrodes out of a plurality of surface electrodes on the maternal patient's abdomen. At step 2502, EMG signals are obtained from the maternal patient via the plurality of surface electrodes, for example after completion of the first plurality of stimulation pulses and prior to generating a second plurality of stimulation pulses. The EMG signals may be obtained via a different subset of the plurality of surface electrodes on the maternal patient's abdomen. Alternatively, the EMG signals may be obtained via the first set of electrodes, and thus the same electrodes utilized to deliver the first plurality of stimulation pulses. At step 2503, a stimulation response of the uterine muscle to the plurality of stimulation pulses is determined based on the EMG signals. For example, the stimulation response may be determined by the control system 99 based on one or more of the frequency of the EMG signal(s), the amplitude of the EMG signal(s), the contraction duration indicated by the EMG signals, the contraction frequency indicated by the EMG signals, etc.

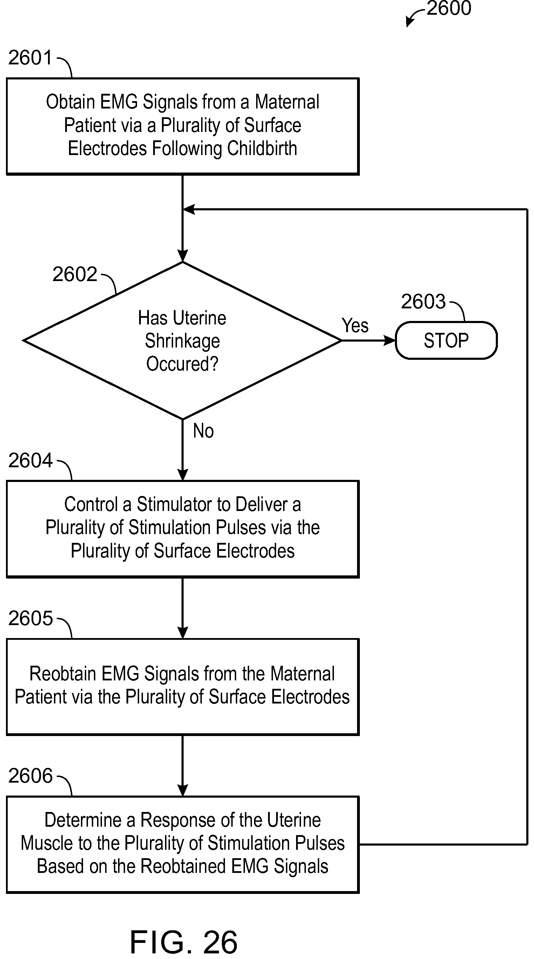

FIG. 26 shows a flow chart embodying an exemplary method 2600 for controlling uterine muscle stimulation of a postpartum maternal patient according to the present disclosure, wherein the postpartum stimulation is configured to cause uterine shrinkage following childbirth. Uterine shrinkage is a normal post-birth biological process by which the uterus returns to its pre-pregnancy size, or significantly decreases in size towards its pre-pregnancy size. A lack of uterine shrinkage is a risk factor for PPH. The inventors have recognized that uterine shrinkage, along with the absence of uterine shrinkage, can be detected via EMG measurements. Certain features of the EMG measurements, such as the peak amplitude, can be assessed as inputs by a computer program configured to determine whether uterine shrinkage has occurred. For example, a peak amplitude of the EMG signal should exceed a threshold value, which is greater than or at least equal to the peak amplitude captured during antepartum and intrapartum contractions. This is because the strength of at least one contraction which causes uterine shrinkage is generally higher than the strength of the contraction which occurs before and during birth.

Alternatively or additionally, the voltage offset of the EMG measurement may also be assessed as inputs by a computer program configured to determine whether uterine shrinkage has occurred. Once the initial uterine shrinkage has occurred, the uterus remains in a shrunken state. This shrunken state is evident in an EMG signal as a voltage offset (a DC shift) from the pre-shrunken state (i.e., due to continual lower-magnitude uterine muscle contractions). This offset can be detected to confirm that initial uterine shrinkage has occurred. If uterine shrinkage has not occurred, the inventors have recognized that surface electrodes on the mother's abdomen, such as the EMG electrodes utilized to collect the EMG signals or other skin electrodes on the maternal abdomen, may be used to deliver stimulation pulses to the uterine muscle with the goal of stimulating uterine contractions to assist the body with initiating uterine shrinkage.

At step 2601, EMG signals are obtained from a maternal patient via a plurality of surface electrodes following childbirth by the maternal patient, wherein the plurality of surface electrodes are skin electrodes configured to be disposed on an abdomen of a maternal patient. At 2602, computer program logic is executed to evaluate whether the uterus has shrunk based on the EMG signals. This may include comparing the response amplitude of the uterine muscle to at least one threshold. For example, this may include comparing an amplitude of the EMG signals to an amplitude threshold determined based on the amplitude of EMG signals recorded while the maternal patient was in labor. If uterine shrinkage has occurred, then the analysis is complete and the method concludes at step 2603. If uterine shrinkage has not occurred, the method proceeds to step 2604 wherein a stimulator is controlled to deliver a plurality of stimulation pulses via the plurality of surface electrodes, The plurality of stimulation pulses is configured to stimulate a uterine muscle of the maternal patient to cause uterine shrinkage.

At step 2605, EMG signals are reobtained from the maternal patient via the plurality of surface electrodes. At step 2606, a response of the uterine muscle to the plurality of stimulation pulses is determined based on the reobtained EMG signals. For example, the response of the uterine muscle may be identified based on a peak amplitude of the reobtained EMG signals, an average amplitude of the reobtained EMG signals (e.g., indicating a DC shift), and/or a frequency of the reobtained EMG signals. A response of the uterine muscle may be compared to at least one threshold. This threshold may be based on the EMG signals obtained from the patient. In some embodiments, the control system may be configured to control delivery of subsequent stimulation based on the identified response, as exemplified and described below.

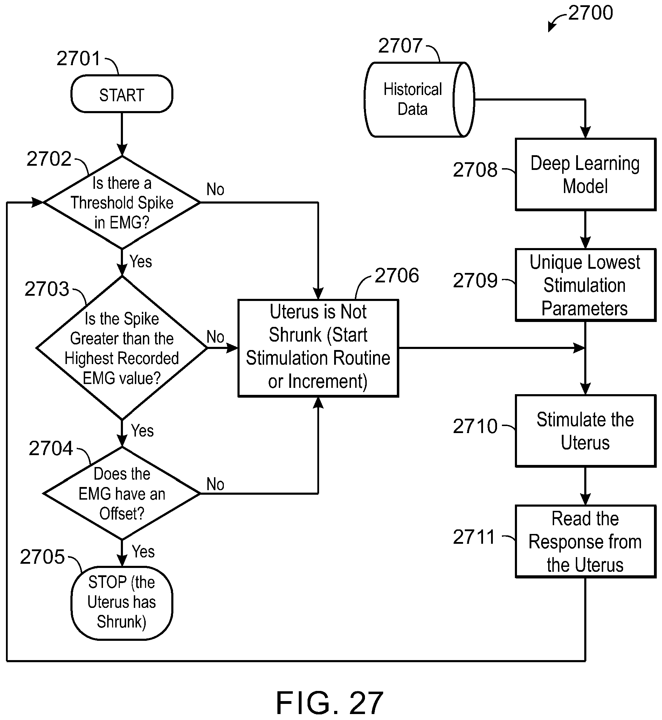

FIG. 27 shows a flow chart embodying a method 2700 according to the present disclosure. The method begins at step 2701. The method may start at a user-selected time or may start automatically. The method is designed to run after a maternal patient has given birth. At step 2702 the method evaluates whether there has been a spike in the amplitude of EMG greater than a predetermined spike threshold. A spike threshold is a threshold change in EMG amplitude recorded over a predetermined amount of time. For example, the predetermined spike threshold may be a patient-specific amplitude value (e.g., change magnitude) determined based on the previously recorded EMG signals for the patient. Alternatively, the predetermined spike threshold may be a predetermined value utilized across all patients or across certain patient populations.

A threshold spike in EMG may indicate that uterine shrinkage has occurred. A lack of a threshold spike indicates that uterine shrinkage has not occurred and the patient is at an elevated risk of PPH. If a threshold spike in the EMG signal has not been detected, the executed method determines that uterine shrinkage has not occurred and proceeds to step 2706, where a stimulation routine is initiated. If a threshold spike in EMG magnitude has been detected in the EMG signal, the executed method determines that uterine shrinkage may have occurred and proceeds to step 2703, where logic is executed by the control system to evaluate whether the spike in EMG amplitude is greater than the highest EMG amplitude value recorded during the antepartum or intrapartum phases of labor. The highest EMG amplitude value recorded during the antepartum or intrapartum phases of labor acts as another threshold value. Typically, the EMG spike detected when uterine shrinkage occurs is greater in magnitude than any EMG magnitude during the antepartum or intrapartum phases of labor. By comparing the threshold EMG spike to the highest EMG value recorded during the antepartum or intrapartum phases of labor, the system further determines whether uterine shrinkage has occurred. If the EMG spike is not greater than the highest EMG value previously recorded during the antepartum or intrapartum phases of labor, the executed method logic may be configured to determine that uterine shrinkage has not occurred and proceeds to step 2706, where the stimulation routine is initiated.

If the EMG spike is greater than the highest EMG value recorded during the antepartum or intrapartum phases of labor the method determines that uterine shrinkage may have occurred and proceeds to step 2704, where the method evaluates whether the EMG signals reflect a DC offset indicating continued muscle contraction. This is because after uterine shrinkage has occurred the EMG signal will be offset from the pre-shrinkage EMG signal due to the continued muscle contractions and shrunken state of the uterus. If a DC offset is not reflected in the EMG signal, the method determines that uterine shrinkage has not occurred and proceeds to step 2706, where the stimulation routine is initiated. If the EMG does have an offset the method proceeds to step 2705, at which the method determines that the uterus of a maternal patient has sufficiently contracted and shrunk and the assessment routine is terminated.

At step 2706 the method determines that the uterus of a maternal patient has not shrunk and it begins the stimulation routine. At step 2707 historical data is fed into a trained deep learning model at 2708. The historical data may be data related to the particular maternal patient in question. The historical data may include EMG measurements from the maternal patient captured during the antepartum and intrapartum phases of labor. The deep learning model 2708 may be a trained neural network trained to calculate stimulation decisions from the uterine atony, placental anomaly, and adenomyosis scores and postpartum EMG measurements. In various embodiments, the deep learning model 2708 may be a machine learning model such as a classification model such as a decision tree, K-nearest neighbor, random forest, a neural network trained via supervised learning on historical and patient data, or a neural network trained via reinforcement learning. The deep learning model 2708 determines the unique lowest stimulation parameters based on the patient-specific inputs and outputs them in step 2709. The unique lowest stimulation parameters may be one or more of a stimulation amplitude, a stimulation frequency, and/or a stimulation pulse width. In some embodiments, the deep learning model 2708 may be configured to determine only a subset of the stimulation parameters as patient-specific parameters and the remaining parameters may be predetermined values.

Also at step 2709, if the method has already run, the increment value (increase amount) is added to one or more of the previously used parameters. The increase amount may be based on a user input, such as by a clinician operating the system or by the maternal patient wearing the system. In another embodiment, the increase amount may be a predetermined amount. In still other embodiments, the control system executing the stimulation control routine may be configured to determine the increase amount, such as the deep learning model 2708 or other model being trained to output an increment amount or by algorithmically determining the increase amount such as based on the magnitude or other values of the EMG signals.

At step 2710 the uterus is stimulated according to the output in step 2709. Utilizing EMG electrodes, the system stimulates a maternal patient's uterus by sending pulses with the characteristics calculated by deep learning model 2708 to the uterine muscle. By stimulating the uterine muscle, the inventors have recognized uterine shrinkage can be encouraged or initiated in a non-invasive manner, thereby decreasing the risk of PPH. At step 2711 the response from the uterus to the stimulation is read via EMG readings. As discussed above, the EMG readings may be obtained with the same EMG electrodes used for stimulation, or may be obtained from different EMG electrodes disposed on the abdomen of a maternal patient. After step 2711 the method returns to steps 2701-2705 to evaluate whether the uterine shrinkage has been sufficiently initiated. If it has not, the method proceeds to step 2706, where the stimulation parameters are incremented by an amount, which may be a predetermined amount, a patient-specific amount, or an operator-defined amount.

This written description uses examples to disclose the invention(s), including the best mode, and also to enable any person skilled in the art to make and use the invention(s). Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention(s) is defined by the claims and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A system for monitoring and stimulating uterine muscles, the system comprising:
a plurality of surface electrodes configured to be disposed on an abdomen of a maternal patient;
a control system configured to:
obtain electromyography (EMG) data via the plurality of surface electrodes;
detect at least one indicator of insufficient uterine activity based on the EMG data; and
generate stimulation pulses between at least two of the plurality of surface electrodes in response to detecting the at least one indicator of insufficient uterine activity.

2. The system of claim 1, wherein the control system is further configured to determine a stimulation frequency, a stimulation amplitude, and a stimulation pulse duration based on the at least one indicator of insufficient uterine activity and/or the EMG data, and to generate the stimulation pulses based on the simulation frequency, the stimulation amplitude, and the pulse duration.

3. The system of claim 1, wherein the stimulation pulses are generated between an upper electrode positioned on an upper portion of the maternal abdomen and a lower electrode positioned on a lower portion of the maternal abdomen.

4. The system of claim 1, wherein the at least one indicator of insufficient uterine activity is based on at least one of a frequency of the EMG data during at least one uterine contraction, an amplitude of the EMG data during the at least one uterine contraction, a duration of the at least one uterine contraction, and a contraction frequency of the at least one uterine contraction.

5. The system of claim 4, wherein the at least one indicator of insufficient uterine activity is based on a comparison of the frequency of the EMG data and the amplitude of the EMG data to one or more frequency thresholds and amplitude thresholds associated with uterus health, a tired uterus condition, a presence of endometrial tissue, and invasive placentation.

6. The system of claim 1, wherein the control system is further configured to detect the at least one indicator of insufficient uterine activity based on a current labor phase of the maternal patient.

7. The system of claim 6, wherein the current labor phase of the maternal patient is an antepartum phase and, wherein the at least one indicator of insufficient uterine activity includes at least one of an EMG frequency being less than at least one antepartum threshold frequency and an EMG amplitude of less than an antepartum threshold amplitude measured during uterine contractions of the maternal patient.

8. The system of claim 6, wherein the current labor phase of the maternal patient is an intrapartum phase and, wherein the at least one indicator of insufficient uterine activity includes at least one of an EMG frequency being less than at least one intrapartum threshold frequency and an EMG amplitude of less than an intrapartum threshold amplitude measured during uterine contractions of the maternal patient.

9. The system of claim 6, wherein the labor phase of the maternal patient is a postpartum phase and, wherein the at least one indicator of insufficient uterine activity includes at least one of an EMG frequency being less than at least one postpartum threshold frequency and an EMG amplitude of less than a postpartum threshold amplitude measured during uterine contractions of the maternal patient.

10. The system of claim 1, further comprising:
an antepartum model being a trained machine learning model stored in memory and trained to detect insufficient uterine activity based on EMG data obtained during antepartum phase of labor;
an intrapartum model being a trained machine learning model stored in memory and trained to detect insufficient uterine activity based on EMG data obtained during intrapartum phase of labor;
a postpartum model being a trained machine learning model stored in memory and trained to detect insufficient uterine activity based on EMG data obtained during postpartum phase of labor; and
wherein the control system is configured to utilize at least one of the antepartum model, the intrapartum model, and the postpartum model to detect the at least one indicator of insufficient uterine activity based on the EMG data.

11. A method of controlling a patient monitoring and stimulation system for a maternal patient, the method comprising:
obtaining electromyography (EMG) data via a plurality of surface electrodes configured to be disposed on an abdomen of a maternal patient;
detecting at least one indicator of insufficient uterine activity based on the EMG data; and
generating stimulation pulses between at least two of the plurality of surface electrodes in response to detecting the at least one indicator of insufficient uterine activity.

12. The method of claim 11**,** wherein the at least one indicator of insufficient uterine activity is based on at least one of a frequency of the EMG data during at least one uterine contraction, an amplitude of the EMG data during the at least one uterine contraction, a duration of the at least one uterine contraction, and a contraction frequency of the at least one uterine contraction, and/or wherein the at least one indicator of insufficient uterine activity includes at least one of an EMG frequency of less than a threshold frequency and an EMG amplitude of less than a threshold amplitude.

13. The method of claim 11, further comprising determining a stimulation frequency, a stimulation amplitude, and a stimulation pulse duration based on the indicator of insufficient uterine activity and/or the EMG data; and
generating a first set of stimulation pulses based on the simulation frequency, the stimulation amplitude, and the pulse duration; and/or
obtaining further EMG data via the plurality of surface electrodes during generation of the stimulation pulses; and/or
detecting a stimulation response based on the EMG data; and/or
determining a stimulation adjustment based on the stimulation response; and/or
generating a second set of stimulation pulses between the at least two of the plurality of surface electrodes based on the stimulation adjustment; and/or
determining that the stimulation response is insufficient based on the EMG data, and wherein the stimulation adjustment includes ceasing stimulation and generating an alert of insufficient response.

14. The method of claim 13, wherein the stimulation adjustment includes at least one of a different stimulation frequency, a different stimulation amplitude, and a different pulse duration from the simulation frequency, the stimulation amplitude, and the pulse duration of the first set of stimulation pulses.

15. The method of claim 11, further comprising: detecting the indicator of insufficient uterine activity based on a current labor phase of the maternal patient, wherein the current labor phase is one of an antepartum phase, an intrapartum phase, and a postpartum phase; and/or
selecting one of an antepartum model, an intrapartum model, and a postpartum model based on the current labor phase of the maternal patient; and/or
utilizing the selected model to detect the at least one indicator of insufficient uterine activity based on the EMG data; and/or
selecting at least one threshold frequency and at least one threshold amplitude based on the
current labor phase of the maternal patient; and/or
detecting the at least one indicator of insufficient uterine activity based on a comparison of the EMG data to the at least one threshold frequency and the at least one threshold amplitude; and/or
wherein the antepartum model is a trained machine learning model stored trained to detect insufficient uterine activity based on EMG data obtained during antepartum phase of labor, the intrapartum model is a trained machine learning model trained to detect insufficient uterine activity based on EMG data obtained during intrapartum phase of labor, and the postpartum model is a trained machine learning model trained to detect insufficient uterine activity based on EMG data obtained during postpartum phase of labor.
